# EUROPEAN PATENT APPLICATION

(11) **EP 1 138 713 A2**
(43) Date of publication of application: **04.10.2001**
(21) Application number: 01302864.2
(22) Date of filing: 28.03.2001
(51) Int. Cl.: C08G 61/12

(54) **Compounds and their manufacture and use**

(30) Priority: 28.03.2000 GB 0007333
(71) Applicant: Sharp Kabushiki Kaisha, Osaka-shi, Osaka 545-8522 (JP)
(72) Inventor: Anderson, Sally, Oxford OX4 1TD (GB); Weaver, Michael Stuart, Warrington, Cheshire WA2 0AB (GB)
(74) Representative: Suckling, Andrew Michael

(57) **Abstract**

Benzofuran, benzothiophene or indole oligomers, co-oligomers, polymers and copolymers, methods for their production, and their use in charge transport and light emission regions of electroluminescent devices are described.

## Description

This invention relates to novel compounds and their use, and is particularly concerned with novel benzofuran, benzothiophene or indole derivatives, methods for their manufacture, and the use of such derivatives in devices such as electroluminescent devices, lasers and thin film transistors, where there is a requirement for materials which are relatively easily manufactured and incorporated into devices as charge transport materials and, potentially, light emission materials.

Organic electroluminescent devices are based on the principle that current injected into an emitter material results in the formation of an energetically excited state. When the material in its excited state decays to its ground state, there is an emission of light. Devices having high emission at low voltages are known which comprise two superimposed layers of organic materials disposed between electrodes. One of the layers is for electron transport and the other is for hole transport. Light emission occurs as a result of electron-hole recombination. It is also known to dope the electron transport layer with a suitable emissive dye at the interface between the layers so that recombination of electrons and holes at the interface causes the dye to emit at high efficiency and with purity of colour.

Organic materials for electroluminescence have, in recent years, been the subject of intensive research. Many different types of materials are known. As disclosed, for example, in J. Mater. Res., Vol. 11, No. 12, December 1996, pages 3174 to 3187, many electron deficient materials have been proposed for electron transport in electroluminescent devices. For example, materials containing an oxadiazole core and derivatives of tris-8-hydroxyquinolinealuminum have been proposed. Hole transport materials are usually based on tertiary amines such as p,p'-di-(N-naphthyl-N-phenyl amine)-biphenyl, commonly called NPB.

US 5 840 217 discloses a wide variety of spiro compounds and their use as electroluminescence materials, in particular spirobifluorene derivatives are disclosed which can have a variety of substituents including phenyl, biphenyl, terphenyl, phenylpropenyl, biphenylpropenyl, benzofuranyl, and benzoxazolyl, inter alia. There may be up to four such substituents, one on each ring of the spirobifluorene moiety.

US 5 077 142 discloses electroluminescent devices in which an aromatic core moiety (such as phenyl, biphenyl, naphthyl or methoxyphenyl) is substituted with a polycyclic hydrocarbon (such as naphthalene, anthracene, naphthacene etc.), an oxygen heterocycle (such as furane, benzofuran, isobenzofuran, pyrrone, isonaphthofuran, etc.), a sulphur heterocycle (such as thiophene, isobenzothiophene, isonaphthothiophene etc.), or a nitrogen heterocycle (such as pyrrole, imidazole, pyridine, pyrimidine, quinoline, triazine, etc).

US 4 900 831 and US 4 948 893 disclose 6-tertiaryaminobenzofuran compounds containing resonant chromophores at the 2-position in the benzofuran ring, for use in organic electroluminescent cells.

EP-A-0545417 discloses conducting polymers, having high stability and exhibiting high solubility in water, which may be used in electronic display elements. The polymers consist of repeating units of the general formula: 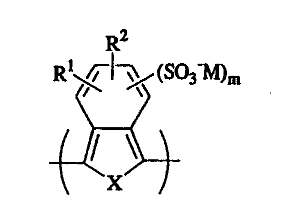 where X represents S, O, Se, Te or NR₃, R₃ represents H, a C₁ to C₆ alkyl group or an aryl group, and M represents a cation such as H⁺, an alkali metal ion or quaternary ammonium. The monomeric unit is substituted containing at least a sulphate group and may also contain up to two more substituted groups which can be alkyl, alkoxy, amino, trihalomethyl or phenyl groups. These polymers are produced by solution polymerisation by the reaction of a sulfonating agent on a derivative of isothianapthene or isobenzofuran for example.

WO 95/09193 discloses polymers formed from bis (2,3-dihydroindole-2,3-dione) compounds, by bulk or solution polymerisation, having the general formula:

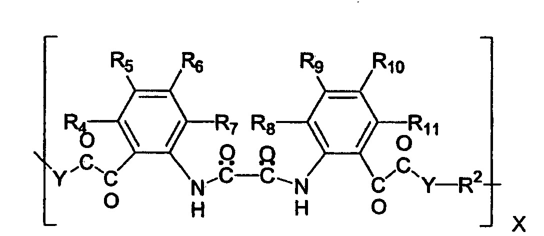

In particular it discloses the use of such polymers as thermoplastic elastomers.

US 6033601 discloses semiconducting organic polymers for use in gas sensors. The polymer is formed by electrochemical polymerisation of 1-substituted, 3-substituted or 1,3 substituted indole monomers.

US 5721333 discloses various soluble polymers of 5,6-dihydroxyindole, and a method for their production by solution polymerisation, for use in cosmetic compositions.

US 5290891 discloses a process for the preparation of polymers based on polyindoles by chemical polymerisation of indole in the presence of an oxidising agent and a solvent. These have high electrical conductivity properties for use in electroconductive devices, display screens and optoelectronic devices.

JP 1156326 discloses a method for the production of an electrically conductive polymer for use as a semiconductor by solution polymerisation of benzothiophene in a solution of a group III metal chloride such as AlCl₃ or FeCl₃.

JP 63307604 discloses a method for the production of electrically conductive polymers from isothianapthene and isoindole by solution polymerisation. These are produced by polymerisation of the repeating units through the heterocyclic ring and have the general formula: 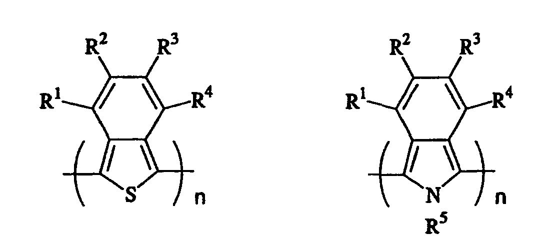 where n is between 5 and 500.

JP 63122727 discloses a method of producing an electrically conductive polymer of the general formula: 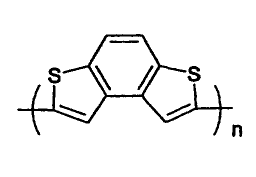 by electrolytic polymerisation of benzodithiophene in the presence of negative ions such tetrafluoroboron ions, perchloric acid ions, hexafluoroarsenic ions, sulphate ions or hydrogen sulphate ions.

JP 61000223 discloses a method of production of a electrically conductive polymer of isobenzothiophene with the phenyl group in the side chain, having excellent oxidation stability, being polymerised in the presence of a halide of at least one type of element selected from B, Si, As, Sb and P.

ES 2092426 discloses soluble electrically conductive copolymers based on indole and a second heterocyclic substance (R) such as thiophene or pyrrole having the general formula: 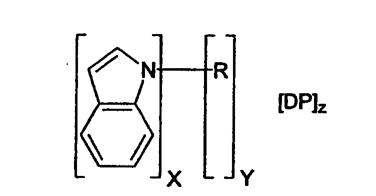 these are produced by solution polymerisation in the presence of an oxidising / doping agent (DP) such as FeCl₃. These copolymers are polymerised through the heterocyclic rings of the repeating units.

It is an object of the present invention to provide a novel class of materials capable of being used for charge transport or emission in EL devices, lasers etc., which are relatively simple to produce in good yield.

According to a first aspect of the present invention, there is provided a substituted or unsubstituted benzofuran, benzothiophene or indole oligomer, co-oligomer, polymer or co-polymer which comprises a unit of the general **formula (I):** 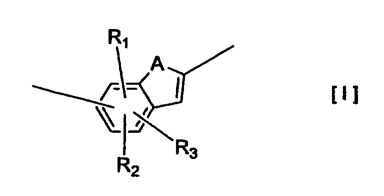 wherein A is O, S or NH, and each of R₁, R₂ and R₃ is independently selected from hydrogen, an aliphatic substituent, and an aromatic substituent, and where the unit recurs at least twice, and in the case of oligomers is preferably 2 to 4.

According to a second aspect of the present invention, there is provided the use of a benzofuran, benzothiophene or indole oligomer, co-oligomer, polymer or co-polymer according to the first aspect in a charge transport region or an electroluminecent region of an electronic device.

According to a third aspect of the present invention, there is provided an electroluminescent structure including a charge transport or electroluminescent region containing a benzofuran, benzothiophene or indole oligomer, co-oligomer, polymer or co-polymer according to the first aspect.

According to a fourth aspect of the present invention, there is provided an electronic device containing a benzofuran, benzothiophene or indole oligomer, co-oligomer, polymer or co-polymer according to the first aspect as a charge transport or photoemission material.

In the case of benzofuran, benzothiophene or indole co-oligomers and co-polymers, these are compounds which contain one benzofuran, benzothiophene or indole-based moiety linked to a different benzofuran, benzothiophene or indole-based moiety, and which typically include the structure (II) below:- 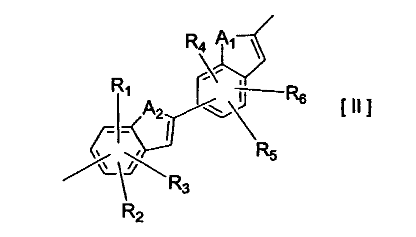 wherein A₁ and A₂ are independently selected from O, S and NH, each of R1, R₂ and R₃ is as defined above, and each of R₄, R₅ and R₆ is independently selected from hydrogen, an aliphatic substituent, and an aromatic substituent, provided that not all of R₄, R₅ and R₆ are the same substituents as R₁, R₂ and R₃ and in the same respective positions as R₁, R₂ and R₃.

The compounds of the present invention are usually conjugated compounds.

The benzofuran, benzothiophene or indole compound may be unsubstituted at at least one of its terminal ends, i.e. it may be hydrogen terminated at one or both ends, or it may be terminated at one or both ends by any desired substituent group. For example, it may be terminated by an unsubstituted benzofuran, benzothiophene or indole moiety at one end, and by a phenyl group at its other end.

The benzofuran, benzothiophene or indole compounds of the present invention can be very conveniently produced by a method involving the use of one or more blocked carbonyloxy-, carbonylthio- or carbonylamino-substituted phenylene ethynylene reactants. Such a reactant or reactants may be oligomerised, co-oligomerised, polymerised or copolymerised before being deprotected and subjected to ring closure to result in conversion of the orthohydroxy(or thio or amino)phenylacetylene into a benzofuran, benzothiophene or indole moiety. Conveniently, the method may be carried out by combinatorial or fast parallel synthesis in solution or on a polymer resin, such as a Merrifield resin.

Typical reaction schemes for producing benzofuran oligomers on a polymer resin in accordance with the present invention are shown below:-

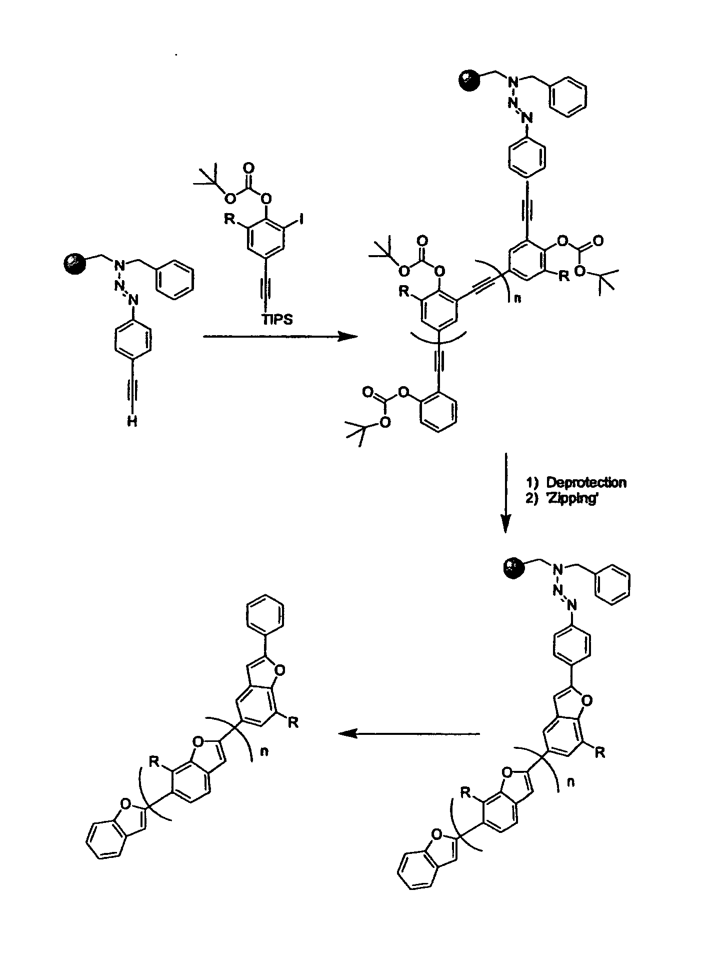

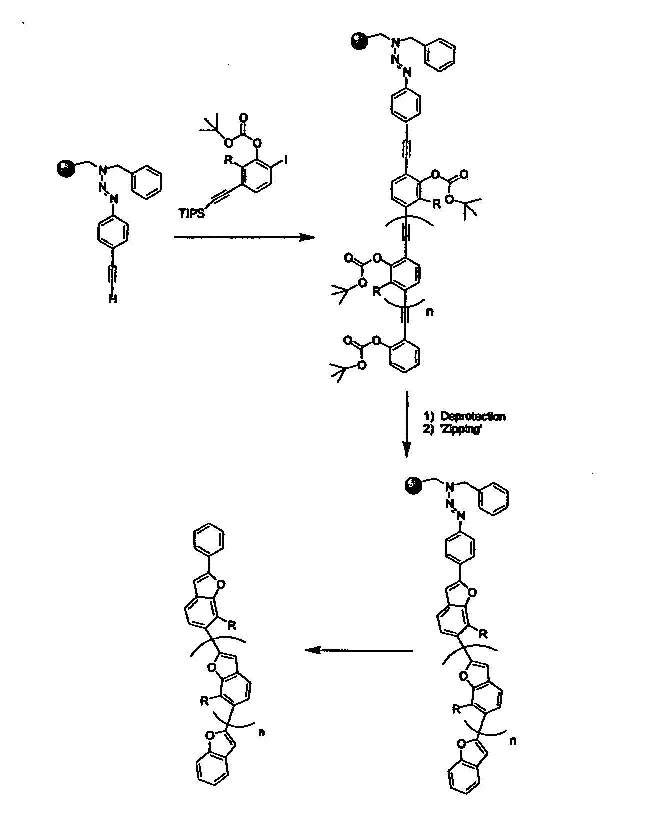

It will be appreciated from the above reaction schemes that different geometries can be obtained in the final product.

A typical method of producing a benzofuran oligomer by synthesis in solution is described below in relation to the preparation of Compound 12, for example.

The corresponding benzothiophene and indole analogues can be producing in similar ways.

The side group R may be readily varied to "tune" the properties of the benzofuran, benzothiophene or indole product.

The length of the oligomer, co-oligomer, polymer or co-polymer may be varied to vary the thermal stability and thin film-forming properties of the material.

The use of combinatorial synthesis is particularly advantageous in that it facilitates the production of a wide variety of benzofuran, benzothiophene or indole oligomers, co-oligomers, polymers/co-polymers which can then be screened (either directly as mixtures or, after separation, as individual compounds) for advantageous charge transport and/or emission properties.

It will further be understood that, when combinatorial synthesis is performed, more than one starting material is employed, so as to produce a mixture of oligomers, co-oligomers, polymers and/or copolymers.

The present invention will now be described in further detail in the following Examples.

### Example 1

### Preparation of tert-Butyl substituted Benzofuran Trimer (Compound 12)

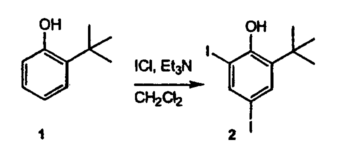

In an analagous manner to the procedures described by T. Marti, B. R. Peterson, A. Fürer, T. Mordasini-Denti, J. Zarske, B. Jaun, F. Diederich and V. Gramlich, (*Hel. Chim. Acta,* **1998,** *81,* 109), triethylamine (23.3 ml, 16.9 g, 0.167 mol) was added to a solution of 2-*tert*-butylphenol (10 g, 10.22 ml, 0.067 mol) in dichloromethane (600 ml) at 0 °C under nitrogen. After slow addition of a solution of iodine monochloride (21.76 g, 0.134 mol) in dichloromethane (200 ml), the dark mixture was stirred for 3.5 h at 0 °C and then quenched by addition of glacial acetic acid (7.0 ml), saturated aqueous sodium thiosulphate solution (300 ml) and water (1000 ml). The separated aqueous layer was extracted with ethylacetate (2 x 500 ml), the combined organic layers were washed with brine (2 x 600 ml), dried (MgSO₄) and the solvent evaporated. The dried product was chromatographed on silica eluting with a 1:1 mixture of dichloromethane and hexane.
Yield 23.35 g, 87 % of **2.**
¹H NMR (300 MHz, CDCl₃) 7.80 (d, J = 2 Hz, 1 H), 7.47 (d, J = 2 Hz, 1 H), 5.51 (s, 1 H), 1.36 (s, 9 H).
¹³C NMR (75 MHz, CDCl₃) 153.17, 143.41, 139.63, 137.11, 90.47, 83.54, 36.01, 29.52.
GC EI-MS 402 [M]⁺

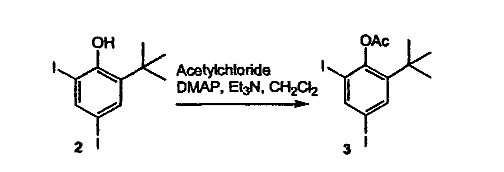

Acetylchloride (2.34 g, 2.12 ml, 2.98 x 10⁻² moles) was added dropwise to a solution of triethylamine (3.02 g, 4.16 ml, 2.98 x 10⁻² moles), diiodophenol **2** (10 g, 0.025 moles) and dimethylaminopyridine (167 mg, 1.49 x 10⁻³moles) in dichloromethane (190 ml) at 0 °C. The mixture was then stirred for 1 h, washed with aqueous ammonium chloride (500 ml, 10 % solution) and aqueous sodium bicarbonate (500 ml, 5 % solution). The organic layer was dried (MgSO₄) and evaporated. The crude product was chromatographed on silica eluting with a 1:3 mixture of dichloromethane and hexane and then recrystallised from a minimum volume of hot ethanol to yield 9.9 g, (90 %) of **3** as a white crystalline solid.
¹H NMR (300 MHz, CDCl₃) 8.02 (d, J = 2 Hz, 1 H), 7.64 (d, J = 2 Hz, 1 H), 2.38 (s, 3 H), 1.30 (s, 9 H).
¹³C NMR (75 MHz, CDCl₃) 168.73, 150.33, 146.07, 145.65, 137.44, 95.97, 91.88, 35.68, 30.68, 22.73.
EI-MS 444 [M]⁺, 402 [M-Ac]⁺

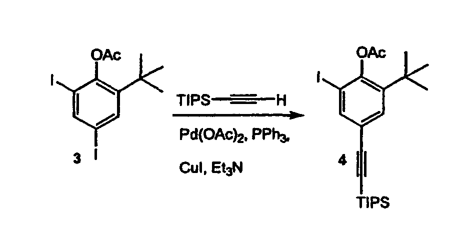

Aryl iodide 3 (7.8 g, 1.76 x 10⁻² moles), palladium(II) acetate (90 mg, 4.0 x 10⁻⁴ moles), copper(I) iodide (38 mg, 2.0 x 10⁻⁴), and triphenylphosphine (210 mg, 8.0 x 10⁻⁴ moles) were dissolved in triethylamine (50 ml, freshly distilled ex. CaH₂), and the mixture degassed using two freeze thaw saturate with nitrogen cycles. Tri*iso*propylsilylacetylene (3.2 g, 3.9 ml, 1.8 x 10⁻² moles) was then added *via* syringe and the mixture degassed by boiling under reduced pressure and then flushing with nitrogen. After three days stirring at room temperature hexanes was added and the triethylamine hydrogen iodide removed *via* filtration through Celite. The filtrate was evaporated and then chromatographed on silica eluting with hexanes containing 2.5 % ethyl acetate, to yield **4** as a white solid 4.68 g, 53 %.
¹H NMR (300 MHz, CDCl₃) 7.81 (d, J = 2 Hz, 1 H), 7.45 (d, J = 2 Hz, 1 H), 2.38 (s, 3 H), 1.32 (s, 9 H), 1.12 (s, 21 H).
¹³C NMR (75 MHz, CDCl₃) 168.85, 150.34, 143.76, 141.30, 131.86, 123.29, 105.38, 94.25, 92.06, 35.55, 30.66, 22.72, 19.07, 11.67.
EI-MS 498 [M]⁺, 445 [M-Ac]⁺

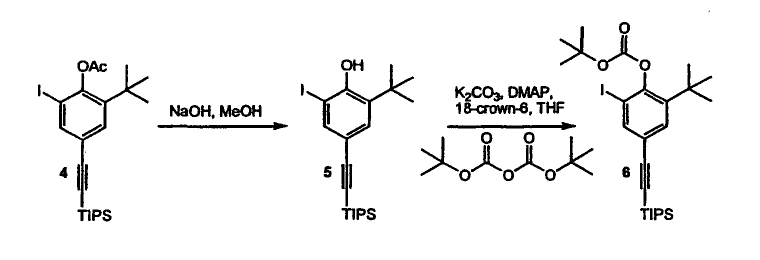

Sodium hydroxide (0.56 g, 1.4 x 10⁻² moles) was dissolved in methanol (2 ml) and added to tri*i*sopropylsilyl protected acetylene **4** (7 g, 1.4 x 10⁻² moles) dissolved in tetrahydrofuran (100 ml). The reaction mixture was left to stir overnight. Further sodium hydroxide (0.56 g, 1.4 x 10⁻² moles in methanol (2ml)) was then added because thin layer chromatography (tlc) revealed that the reaction had not reached completion. When the reaction was complete by tlc, the base was neutralised with hydrochloric acid (10 % aqueous). The mixture was then thoroughly extracted with diethylether. The organic fractions were dried over magnesium sulphate, and then evaporated. The colourless oil was carefully dried under high vacuum (2 x 10⁻² mbar) to yield **5.** All of **5** was then taken on to the next step without further purification or characterisation. Phenol **5,** potassium carbonate (2.93 g, 2.2 x 10⁻² moles), dimethylaminopyridine (catalytic amount) and 18-crown-6 (catalytic amount) were dried under vacuum and then flushed with nitrogen. Tetrahydrofuran (85 ml, dry and oxygen free) was then added *via* syringe followed by BOC-anhydride (3.52 g, 3.7 ml, 1.61 x10⁻² moles). The reaction was then left to stir until no starting material was observed by tlc (1 hour). The reaction was quenched by the addition of brine and the resulting mixture extracted with diethylether. The organic fractions were then dried over magnesium sulphate and evaporated. The pale yellow oil was chromatographed on silica eluting with dichloromethane and hexanes (1:3) to yield 7.3 g, 82 % of **6** as a colourless oil.
¹H NMR (300 MHz, CDCl₃) 7.81 (d, J = 2 Hz, 1 H), 7.43 (d, J = 2 Hz, 1 H), 1.57 (s, 9 H), 1.34 (s, 9 H), 1.12 (s, 21 H).
¹³C NMR (75 MHz, CDCl₃) 150.36, 150.17, 143.82, 141.30, 131.79, 123.20, 105.45, 94.23, 91.96, 84.54, 35.64, 30.57, 28.19, 19.08, 11.68.
CI-MS 574 [M+NH₃]⁺, 457 [M - tBOC]⁺

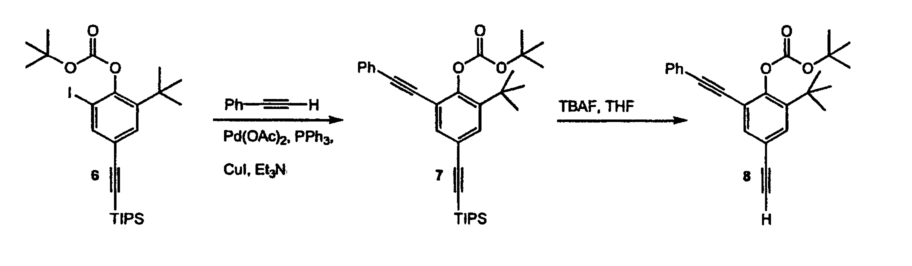

Aryl iodide **6** (1.9 g, 3.42 x 10⁻³ moles), palladium(II) acetate (15.4 mg, 6.8 x 10⁻⁵ moles), copper(I) iodide (6.5 mg, 3.4 x 10⁻⁵ moles), and triphenylphosphine (36 mg, 1.4 x 10⁻⁴ moles) were dissolved in triethylamine (20 ml, freshly distilled ex. CaH₂) and the resulting mixture degassed using two freeze thaw saturate with nitrogen cycles. Phenylacetylene (384 mg, 413 µl, 3.8 x 10⁻³ moles) was added *via* syringe and the resulting solution degassed by boiling under reduced pressure and saturating with nitrogen. The reaction mixture was heated to 70 °C for 6 hours. The reaction mixture was then filtered through Celite, the Celite was washed with hexanes and then the solvent evaporated. Chromatography was carried out on silica eluting with dichloromethane and hexanes (1:3). The resulting colourless oil **7** (1.77 g, 98 %), was then taken on to the next step without further purification or characterisation. To a solution of triisopropyl protected acetylene **7** (1.5 g, 2.83 x 10⁻³ moles) dissolved in dichloromethane tetrabutylammonium fluoride (1 M in THF, 3.11 ml, 3.11 x 10⁻³ moles) was added. The reaction was complete after 15 minutes stirring at room temperature. The reaction was quenched by the addition of calcium chloride and brine, the product was extracted with dichloromethane, the organic fractions were dried over magnesium sulphate and then the solvent evaporated. Chromatography on silica eluting with dichloromethane : hexanes (1:1) yielded 0.82 g, 77 % of **8** as a thick colourless oil.
¹H NMR (300 MHz, CDCl₃) 7.57 (d, J = 2 Hz, 1 H), 7.54-7.51 (m, 2 H), 7.49 (d, J = 2 Hz, 1 H), 7.36-7.32 (m, 3 H), 3.06 (s, 1 H), 1.54 (s, 9 H), 1.38 (s, 9 H).
¹³C NMR (75 MHz, CDCl₃) 151.24, 150.96, 142.88, 134.77, 132.13, 131.77, 129.02, 128.69, 123.21, 119.95, 119.88, 95.02, 84.31, 84.21, 83.14, *35.23,* 30.49, 28.03.
CI-MS 392 [M+NH₃]⁺, 292 [M+NH₃ - tBOC]⁺, 275 [M - tBOC]⁺

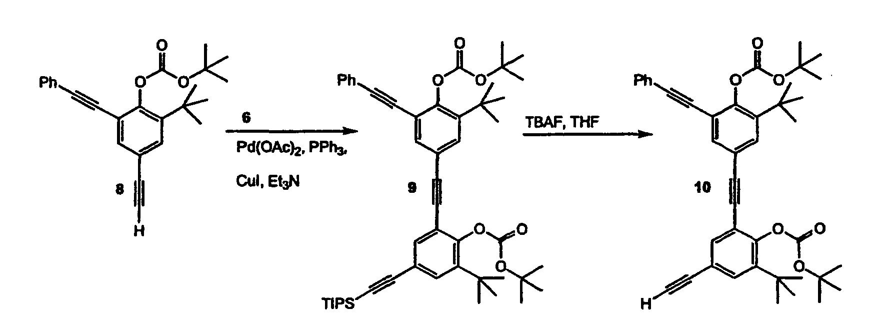

Aryliodide **4** (1.16 g, 2.1 x 10⁻³ moles), arylacetylene **8** (0.78 g, 2.1 x 10⁻³ moles), palladium(II) acetate (9.4 mg, 4.2 x 10⁻⁵ moles), copper(I) iodide (4 mg, 2.1 x 10⁻⁵ moles) and triphenylphosphine (22 mg, 8.4 x 10⁻⁵ moles) were dissolved in triethylamine (15 ml, freshly distilled ex. CaH₂) and the resulting mixture degassed by two freeze thaw saturate with nitrogen cycles. The reaction mixture was stirred at 70 °C for 6 hours, by which time no starting materials were visible by tlc. The reaction mixture was filtered through Celite, the Celite being carefully washed with hexanes. The solvent was then evaporated and the resulting pale yellow oil chromatographed on silica eluting with a mixture of hexanes and dichloromethane (1:1). The resulting colourless oil **9** was dissolved in dichloromethane (50 ml) and tetrabutylammonium fluoride (2.1 ml, 2.1 x 10⁻³ moles) added. The reaction was quenched by the addition of calcium chloride and brine, the product was extracted with dichloromethane, the organic fractions were dried over magnesium sulphate and then the solvent evaporated. Chromatography on silica eluting with a mixture of dichloromethane and hexanes (1:1) yielded **10** (1.29 g, 91 %) as a pale yellow oil.
¹H NMR (300 MHz, CDCl₃) 7.61-7.49 (m, 6 H), 7.36-7.33 (m, 3 H), 1.48 (s, 9 H), 1.46 (s, 9 H), 1.39 (s, 9 H), 1.38 (s, 9 H).
¹³C NMR (75 MHz, CDCl₃) 151.27, 151.09, 150.98, 150.89, 142.93, 142.86, 134.77, 134.49, 132.11, 131.93, 131.20, 129.02, 128.71, 123.24, 120.65, 120.01, 119.87, 119.66, 95.02, 94.08, 84.37, 84.34, 84.21*, 83.08, 77.67, 35.31, 35.25, 30.55, 30.49, 28.08, 28.03. (* indicates two overlapping signals)
CI-MS 664 [M+NH₃]⁺, 564 [M+NH₃ - tBOC]⁺, 464 [M+NH₃ - 2tBOC]⁺

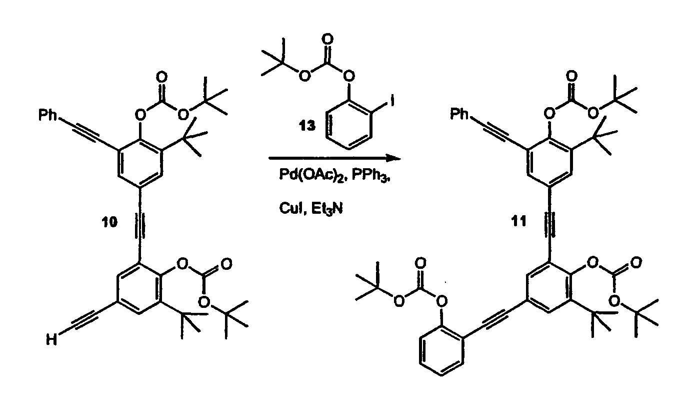

Aryl iodide **13** (0.54 g, 1.7 x 10⁻³ moles), arylacetylene **10** (1.1 g, 1.7 x 10⁻³ moles), palladium(II) acetate (7.6 mg, 3.4 x 10⁻⁵ moles), copper(I) iodide (3.2 mg, 1.7 x 10⁻⁵ moles) and triphenylphosphine (17.9 mg, 6.8 x 10⁻⁵ moles) were dissolved in triethylamine (10 ml, freshly distilled ex CaH₂) and the resulting mixture degassed using two freeze thaw saturate with nitrogen cycles. The reaction mixture was stirred at 70 °C overnight. When the reaction was complete by tlc the reaction mixture was filtered through Celite, the Celite was carefully washed with hexanes and then the solvent evaporated to yield a yellow oil. This oil was chromatographed on silica eluting with a mixture of dichloromethane and hexanes (1:1) to yield **11**, (1.14 g, 80 %) as a white foam.
¹H NMR (300 MHz, CDCl₃) 7.62-7.51 (m, 7 H), 7.40-7.34 (m, 4 H), 7.27-7.19 (m, 2 H), 1.54 (s, 9 H), 1.46 (s, 9 H), 1.39 (s, 18 H).
¹³C NMR (75 MHz, CDCl₃) 152.10, 151.78, 151.08, 151.04, 150.96, 150.92, 142.90, 142.88, 134.45*, 133.43, 132.11, 131.29, 131.20, 130.08, 129.03, 128.71, 126.43, 123.25, 122.50, 120.92, 120.71, 119.89, 119.66, 117.79, 95.01, 94.04, 93.89, 84.49*, 84.38, 84.31, 84.25, 84.22, 35.30*, 30.55*, 28.12, 28.09. (* indicates two overlapping signals)
CI-MS 857 [M+NH₃]⁺, 756 [M+NH₃ - tBOC]⁺, 656 [M+NH₃ - 2tBOC]⁺, 539 [M+NH₃ - 3tBOC]⁺

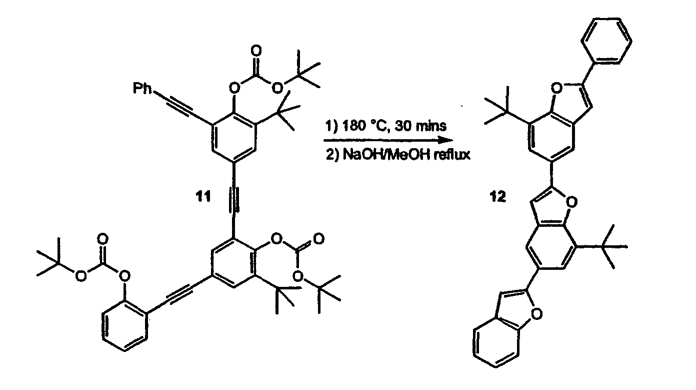

**11** (300 mg, 3.57 x 10⁻⁴ moles) was heated to 180 °C under reduced pressure (0.02 mbar) until no further evolution of gas was observed. The resultant yellow glass was dissolved in methanol (50 ml) and sodium hydroxide (0.051 g, 1.28 x 10⁻³ moles) was added, the resultant mixture was brought to reflux. After refluxing overnight a precipitate had formed which was collected *via* centrifugation and washed with methanol (3 x 50 ml). The white solid was dried under reduced pressure (0.02 mbar, 70 °C). Yield of **12** (120 mg, 63 %)
¹H NMR (300 MHz, CDCl₃) 7.98 (d, J=1.7 Hz, 1 H), 7.96 (d, J=1.6 Hz, 1 H), 7.92-7.89 (m, 2 H), 7.74 (d, J = 1.6 Hz, 1 H), 7.70 (d, J=1.6 Hz,1 H), 7.62-7.52 (m, 2 H), 7.52-7.42 (m, 2H), 7.42-7.30 (m, 1 H), 7.11 (s, 1 H), 7.04 (s, 1 H), 7.02 (d, J=0.6 Hz, 1 H)
EI-MS 538 [M]⁺

Elemental Analysis C:84.69%, H:6.27%.

Quantum efficiency [cyclohexane measured relative to anthracene (0.26)] 0.6 ±0.2

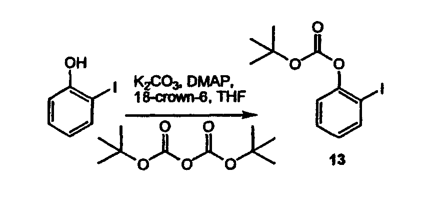

BOC-anhydride (5.4 g, 2.5 x 10⁻² moles) was added to a mixture of 2-iodophenol (5 g, 2.3 x 10⁻² moles), potassium carbonate (4.5 g, 3.4 x 10⁻² moles), dimethylaminopyridine (catalytic amount) and 18-crown-6 (catalytic amount) in tetrahydrofuran (130 ml). After 1 h stirring at room temperature the reaction appeared to be complete by tlc. The reaction was quenched by the addition of brine and the resulting mixture extracted with diethylether. The organic fractions were then dried over magnesium sulphate and evaporated. The pale yellow oil was chromatographed on silica eluting with dichloromethane and hexanes (1:3) to yield 6.9 g, 95 % of **13** as a colourless oil.
¹H NMR (300 MHz, CDCl₃) 7.82 (dd, J=1, 8 Hz, 1 H), 7.36 (ddd, J=1, 8, 8 Hz, 1 H), 7.17 (dd, J=1, 8 Hz, 1H), 6.97 (ddd, J=1, 8, 8 Hz, 1 H), 1.58 (s, 9 H).
¹³C NMR (75 MHz, CDCl₃) 151.77, 151.36, 139.87, 129.94, 128.09, 123.25, 91.03, 84.58, 23.31.
CI-MS 538 [M+NH₃]⁺

### Example 2

### Preparation of the Hexyl substituted Benzofuran Trimer (Compound 26).

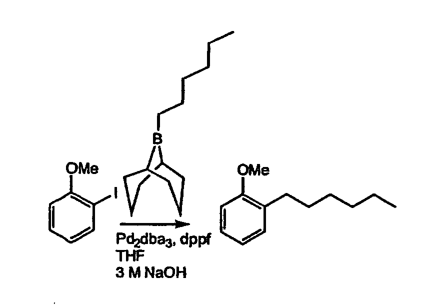

1-hexene (6.9 g, 10.2 ml, 8.2 x 10⁻² moles) and 9-BBN (165 ml, 0.5 M solution in tetrahydrofuran) were mixed at 0 °C and then the mixture was allowed to warm to room temperature overnight. To this mixture was added palladium(0)dibenzylideneacetone (1.0 g, 1.1 x 10⁻³ moles), 1,1-bis(diphenylphosphino)ferrocene (1.2 g, 2.2 x 10⁻³ moles) in tetrahydrofuran (180 ml) containing 3 M sodium hydroxide solution (75 ml) and 2-iodomethoxybenzene (17.23 g, 10 ml, 7.4 x 10⁻² moles), and the resultant mixture left at reflux overnight. Any residual borane was then quenched by the addition of hydrogen peroxide (30 ml). The product was then extracted with hexanes, washed with brine and dried over magnesium sulphate. The solvent was evaporated to yield a colourless oil which was purified by flash column chromatography on silica eluting with a mixture of dichloromethane and hexanes (1:1) to yield 12.17 g, 86 % of 2-hexylmethoxybenzene.
¹H NMR (300 MHz, CDCl₃) 7.2-7.1 (m, 2 H), 6.9-6.8 (m, 2 H), 3.82 (s, 3 H), 2.6-2.5 (m, 2 H), 1.6-1.5 (m, 3 H), 1.4-1.3 (m, 6 H), 1.0-0.8 (m, 3 H).
¹³C NMR (75 MHz, CDCl₃) 157.83, 131.77, 130.12, 127.12, 120.68, 110.59, 55.65, 32.19, 30.55, 30.24, 29.71, 23.07, 14.54.

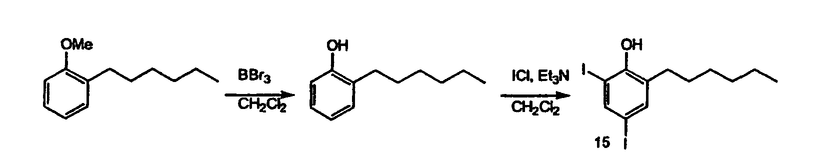

2-Hexylmethoxybenzene (11g, 5.7 x 10⁻² moles) was dissolved in dichloromethane (200 ml) and the mixture degassed by boiling under reduced pressure and then saturating with nitrogen twice, then boron tribromide (21.5 g, 8.6 x 10⁻² moles) was added carefully *via* syringe, and the mixture left to stir under nitrogen. When the reaction was complete the excess boron tribromide was quenched with methanol, and then water. The aqueous layer was neutralised with sodium hydroxide and then extracted with dichloromethane. The dichloromethane fractions were combined and the solvent evaporated to yield a pale brown oil, which was taken on to the next step without further purification. To a solution of 2-hexylphenol **15** (10 g, 5.6 x 10⁻² moles) in dichloromethane (500 ml) was added at 0 °C triethylamine (19.5 ml, 14.2 g, 0.14 moles) under nitrogen. After slow addition of a solution of iodine monochloride (18.2 g, 0.11 moles) in dichloromethane (150 ml), the dark mixture was stirred for 3.5 h at 0 °C and then quenched by addition of glacial acetic acid (6 ml), saturated aqueous sodium thiosulphate solution (250 ml) and water (800 ml). The separated aqueous layer was extracted with ethylacetate (2 x 400 ml), the combined organic layers were washed with brine (2 x 500 ml), dried (MgSO₄) and the solvent evaporated. The dried product was chromatographed on silica eluting with a 1:3 mixture of dichloromethane and hexane to yield 17.3 g, 72 % of **15.**
¹H NMR (300 MHz, CDCl₃) 7.75 (d, J=2 Hz, 1 H), 7.36 (d, J=2 Hz, 1 H), 5.27 (s, 1 H), 2.7-2.5 (m, 2 H), 1,6-1.5 (m, 2 H), 1.4-1.2 (m, 6 H), 1.0-0.8 (m, 3 H).
¹³C NMR (75 MHz, CDCl₃) 153.03, 143.00, 139.59, 132.53, 87.82, 83.32, 32.06, 31.47, 29.76, 29.50, 23.00, 14.54.
GC EI-MS 430 [M]⁺

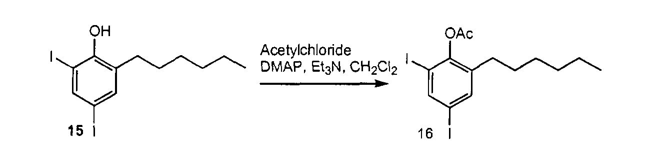

Acetylchloride (3.28 g, 3.0 ml, 4.2 x 10⁻² moles) was added dropwise to a solution of triethylamine (4.22 g, 5.82 ml, 4.18 x 10⁻² moles), **15** (15 g, 3.5 x 10⁻² moles) and dimethyaminopyridine (234 mg, 2.08 x 10⁻³moles) in dichloromethane (250 ml) at 0 °C. The mixture was stirred for 1 h, washed with aqueous ammonium chloride (750 ml, 10 % solution) and aqueous sodium bicarbonate (750 ml, 5 % solution). The organic layer was dried (MgSO₄) and evaporated. The crude product was chromatographed on silica eluting with a 1:1 mixture of dichloromethane and hexane and then recrystallised from a minimum volume of hot ethanol to yield **16** (15.89 g, 85 %).
¹H NMR (300 MHz, CDCl₃) 7.96 (d, J=2 Hz, 1H), 7.51 (d, J=2 Hz, 1 H), 2.5-2.4 (m, 2 H), 2.37 (s, 3 H), 1.6-1.4 (m, 2 H), 1.3 (br.s, 6 H), 0.9-0.8 (m, 3 H).
¹³C NMR (75 MHz, CDCl₃) 168.49, 150.09, 144.82, 139.45, 139.13, 93.36, 91.65, 31.90, 31.34, 29.88, 29.43, 22.92, 21.50, 14.47.
GC EI-MS 472 [M]⁺, 430 [M - Ac]⁺

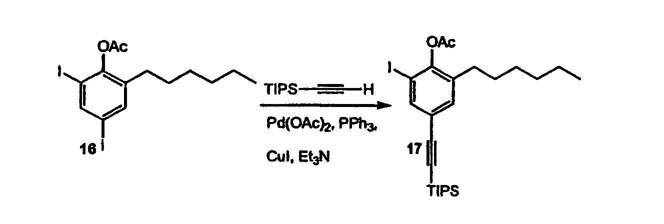

Aryl iodide 16 (7 g, 1.48 x 10⁻² moles), palladium(II) acetate (67 mg, 3.0 x 10⁻⁴ moles), copper(I) iodide (28 mg, 1.5 x 10⁻⁴), and triphenylphosphine (156 mg, 5.9 x 10⁻⁴ moles) were dissolved in triethylamine (40 ml, freshly distilled ex. CaH₂), and the mixture degassed using two freeze thaw saturate with nitrogen cycles. Tri*iso*propylsilylacetylene (2.7 g, 3.3 ml, 1.5 x 10⁻² moles) was then added *via* syringe and the mixture degassed by boiling under reduced pressure and then flushing with nitrogen. After three days stirring at room temperature, hexanes was added and the triethylamine hydrogen iodide removed *via* filtration through Celite. The filtrate was evaporated and then chromatographed on silica eluting with hexanes containing 2.5 % ethyl acetate, to yield 4 as a white solid 5.4 g, 69 %.
¹H NMR (300 MHz, CDCl₃) 7.76 (d, J=2 Hz, 1 H), 7.29 (d, J=2 Hz, 1 H), 2.5-2.4 (m, 2 H), 2.37 (s, 3 H), 1.6-1.5 (m, 2 H), 1.4-1.2 (m, 6 H), 1.12 (s, 21 H), 0.9-0.8 (m, 3 H).
¹³C NMR (75 MHz, CDCl₃) 168.57, 150.07, 140.52, 136.80, 134.10, 123.69, 105.13, 92.20, 91.65, 31.92, 31.52, 29.97, 29.50, 22.94, 21.52, 19.05, 14.46, 11.66.
EI-MS 526 [M]⁺, 483 [M - Ac]⁺

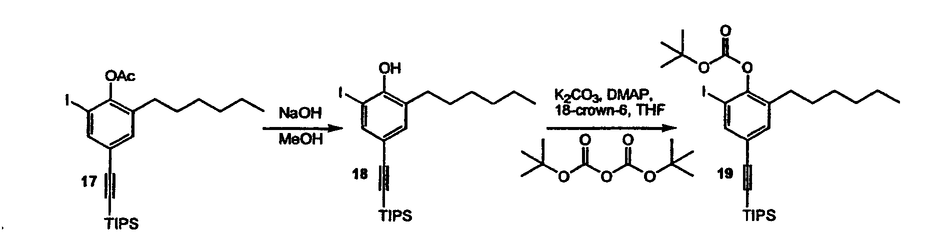

Sodium hydroxide (0.38 g, 9.5 x 10⁻³ moles) was dissolved in methanol (10 ml) and added to triisopropylsilyl protected acetylene **17** (5 g, 9.5 x 10⁻³ moles) dissolved in tetrahydrofuran (100 ml). The reaction mixture was left to stir over night. When the reaction was complete by tlc, the base was neutralised with hydrochloric acid (10 % aqueous). The mixture was then thoroughly extracted with diethylether. The organic fractions were dried over magnesium sulphate, and then evaporated. The colourless oil was carefully dried under high vacuum (2 x 10⁻² mbar) to yield **18.** All of **18** was then taken on to the next step without further purification or characterisation. Phenol **18,** potassium carbonate (1.88 g, 1.36 x 10⁻² moles), dimethylaminopyridine (catalytic amount) and 18-crown-6 (catalytic amount) were dried under vacuum and then flushed with nitrogen. Tetrahydrofuran (50 ml, dry and oxygen free) was then added *via* syringe followed by BOC-anhydride (2.28 g, 2.4 ml, 1.04 x10⁻² moles). The reaction was then left to stir until no starting material was observed by tlc (1 hour). The reaction was quenched by the addition of brine and the resulting mixture extracted with diethylether. The organic fractions were then dried over magnesium sulphate and evaporated. The pale yellow oil was chromatographed on silica eluting with dichloromethane and hexanes (1:3) to yield 5.4 g, 97 % of **19** as a colourless oil.
¹H NMR (300 MHz, CDCl₃) 7.75 (d, J = 2 Hz, 1 H), 7.28 (d, J = 2 Hz, 1 H), 2.55-2.49 (m, 2 H), 1.57 (s, 9 H), 1.57-1.52 (m, 2 H), 1.37-1.25 (m, 6 H), 1.11 (s, 21 H), 0.91-0.85 (m, 3 H).
¹³C NMR (75 MHz, CDCl₃) 150.61, 149.89, 140.56, 136.90, 134.23, 123.58, 105.13, 92.15, 91.69, 84.56, 31.95, 31.39, 30.09, 29.53, 28.09, 22.94, 19.06, 14.51, 11.65.
CI-MS 602 [M+NH₃]⁺, 485 [M - tBOC]⁺

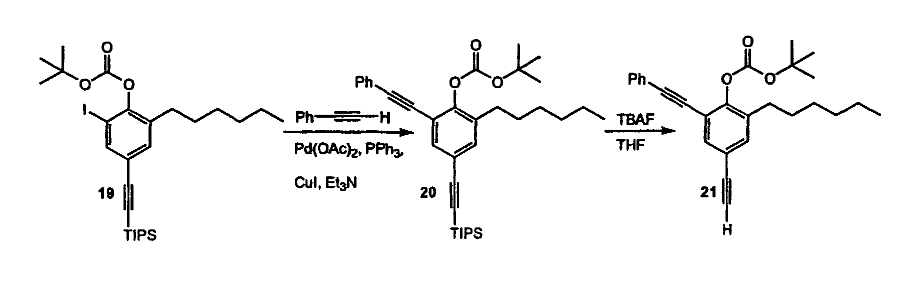

Aryl iodide **19** (2.0 g, 3.42 x 10⁻³ moles), palladium(II) acetate (15.4 mg, 6.8 x 10⁻⁵ moles), copper(I) iodide (6.5 mg, 3.4 x 10⁻⁵ moles), and triphenylphosphine (36 mg, 1.4 x 10⁻⁴ moles) were dissolved in triethylamine (20 ml, freshly distilled ex. CaH₂) and the resulting mixture degassed using two freeze thaw saturate with nitrogen cycles. Phenylacetylene (384 mg, 413 µl, 3.8 x 10⁻³ moles) was added *via* syringe and the resulting solution degassed by boiling under reduced pressure and saturating with nitrogen. The reaction mixture was heated to 70 °C for 6 hours. The reaction mixture was then filtered through Celite, the Celite was washed with hexanes and then the solvent evaporated. Chromatography was carried out on silica eluting with dichloromethane and hexanes (1:3). The resulting colourless oil **20** was then taken on to the next step without further purification or characterisation. To a solution of triisopropyl protected acetylene **20** dissolved in dichloromethane (100 ml) tetrabutylammonium fluoride (1 M in THF, 3.42 ml, 3.42 x 10⁻³ moles) was added. The reaction was complete after 15 minutes stirring at room temperature. The reaction was quenched by the addition of calcium chloride and brine, the product was extracted with dichloromethane, the organic fractions were dried over magnesium sulphate and then the solvent evaporated. Chromatography on silica eluting with dichloromethane : hexanes (1:1) yielded 1.25 g, 91 % of **21** as a thick colourless oil.
¹H NMR (300 MHz, CDCl₃) 7.6-7.4 (m, 3 H), 7.4-7.3 (m, 4 H), 3.06 (s, 1 H), 2.56 (t, J = 7.6 Hz, 2 H), 1.65-1.5 (m, 2 H), 1.49 (s, 9 H), 1.2-1.4 (m, 6 H), 0.89 (t, J = 7 Hz, 3 H).
¹³C NMR (75 MHz, CDCl₃) 151.17, 150.54, 136.29, 134.45, 134.28, 132.10, 129.05, 128.71, 123.20, 120.30, 118.64, 95.02, 84.22, 83.96, 82.80, 77.78, 32.00, 30.44, 30.05, 29.43, 27.99, 22.93, 14.49.
CI-MS 420 [M+NH₃]⁺, 320 [M+NH₃ - tBOC]⁺

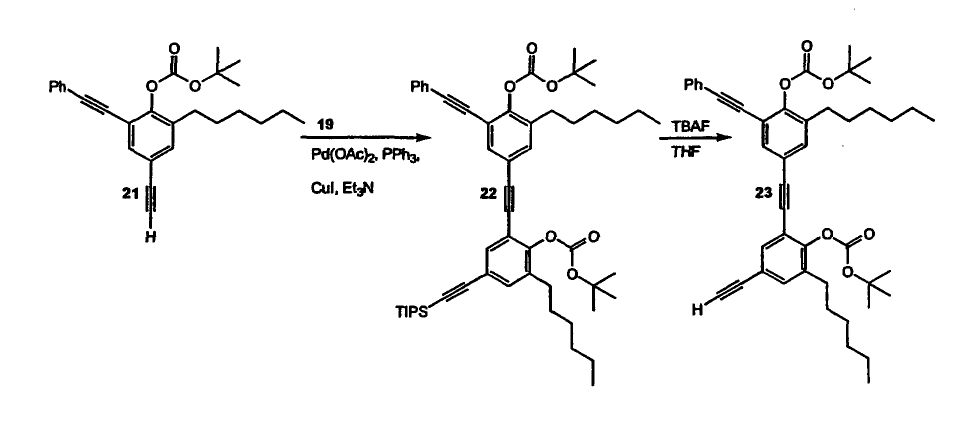

Aryliodide **19** (1.82 g, 3.1 x 10⁻³ moles), arylacetylene **21** (1.25 g, 3.1 x 10⁻³ moles), palladium(II) acetate (13.9 mg, 6.2 x 10⁻⁵ moles), copper(I) iodide (6 mg, 3.1 x 10⁻⁵ moles) and triphenylphosphine (33 mg, 1.2 x 10⁻⁴ moles) were dissolved in triethylamine (20 ml, freshly distilled ex. CaH₂) and the resulting mixture degassed by two freeze thaw saturate with nitrogen cycles. The reaction mixture was stirred at 70 °C for 6 hours, by which time no starting materials were visible by tlc. The reaction mixture was filtered through Celite, the Celite being carefully washed with hexanes. The solvent was then evaporated and the resulting pale yellow oil chromatographed on silica eluting with a mixture of hexanes and dichloromethane (1:1). The resulting colourless oil **22** was dissolved in dichloromethane (125 ml) and tetrabutylammonium fluoride (3.1 ml, 3.1 x 10⁻³ moles) added. The reaction was quenched by the addition of calcium chloride and brine, the product was extracted with dichloromethane, the organic fractions were dried over magnesium sulphate and then the solvent evaporated. Chromatography on silica eluting with a mixture of dichloromethane and hexanes (1:1) yielded **23** (1.96 g, 90 %) as a pale yellow viscous oil.
¹H NMR (300 MHz, CDCl₃) 7.60-7.55 (m, 1 H), 7.55-7.45 (m, 3 H), 7.40-7.30 (m, 5 H), 3.06 (s, 1 H), 2.50-2.64 (m, 4 H), 1.65-1.45 (m, 4 H), 1.40-1.25 (m, 12 H), 0.95-0.80 (m, 6 H).
¹³C NMR (75 MHz, CDCl₃) 151.19, 151.14, 150.58, 150.40, 136.37, 136.29, 134.46, 134.39, 134.10, 133.72, 132.09, 129.05, 128.73, 123.23, 120.97, 120.35, 118.66, 118.39, 95.01, 93.79, 84.35, 84.23, 84.18, 83.99, 82.74, 77.63⁺, 32.03, 32.00, 30.51, 30.44, 30.11, 30.05, 29.46, 29.43, 28.03, 28.00, 22.93*, 14.52*. (* indicates two overlapping signals) (⁺ indicates under CDCl₃)

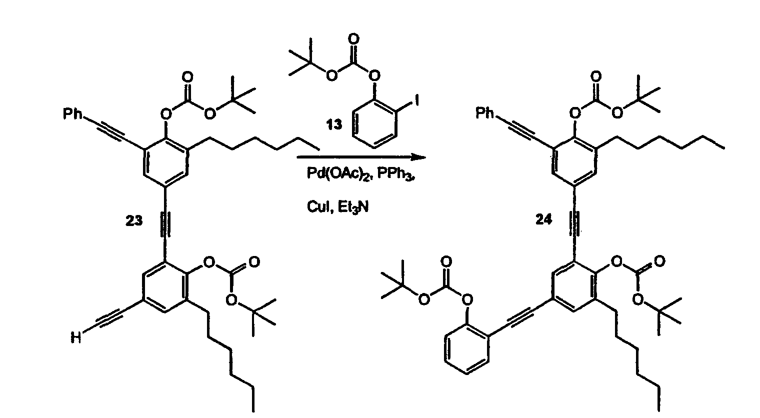

Aryl iodide **13** (0.89 g, 2.8 x 10⁻³ moles), arylacetylene **23** (2.0 g, 2.8 x 10⁻³ moles), palladium(II) acetate (12.5 mg, 5.6 x 10⁻⁵ moles), copper(I) iodide (5.3 mg, 2.8 x 10⁻⁵ moles) and triphenylphosphine (29.3 mg, 1.1 x 10⁻⁴ moles) were dissolved in triethylamine (16 ml, freshly distilled ex CaH₂) and the resulting mixture degassed using two freeze thaw saturate with nitrogen cycles. The reaction mixture was stirred at 70 °C overnight. When the reaction was complete by tlc the reaction mixture was filtered through Celite, the Celite was carefully washed with hexanes and then the solvent evaporated to yield a yellow oil. This oil was chromatographed on silica eluting with a mixture of dichloromethane and hexanes (1:1) to yield **24**, (2.3 g, 92 %) as a white foam.
¹H NMR (300 MHz, CDCl₃) 7.60-7.45 (m, 5 H), 7.40-7.30 (m, 6 H), 7.30-7.15 (m, 2 H), 2.58 (m, 4 H), 1.70-1.45 (m, 4 H), 1.56 (s, 9 H), 1.52 (s, 9 H), 1.50 (s, 9 H), 1.45-1.25 (m, 12 H), 0.85-0.95 (m, 6 H).
¹³C NMR (75 MHz, CDCl₃) 152.14, 151.78, 151.19, 151.16, 150.39, 150.37, 136.33, 136.30, 134.06, 134.03, 133.86, 133.71, 133.37, 132.09, 131.98, 130.09, 129.05, 128.73, 126.42, 123.24, 122.51, 121.25, 121.02, 118.68, 118.39, 117.78, 95.01, 93.73, 93.69, 84.69, 84.30*, 84.25, 84.22, 84.01, 32.03*, 30.51*, 30.11, 30.10, 29.46, 28.11, 28.05, 28.00, 22.92*, 14.50*. (* indicates two overlapping signals)
APCI-MS 794 [M - tBOC]⁺, 695 [M - 2tBOC]⁺, 595 [M-3tBOC]⁺

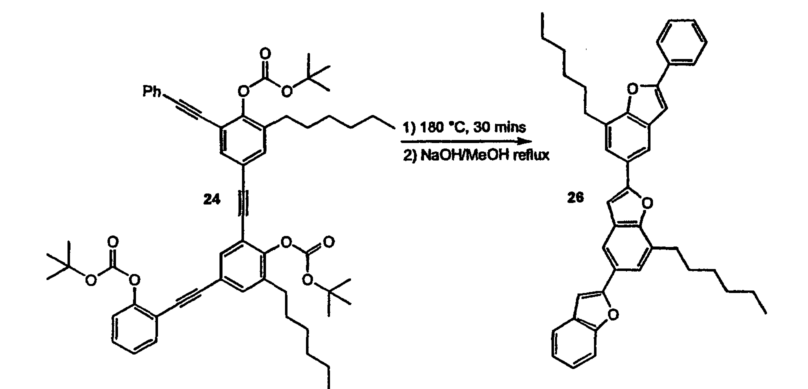

**24** (1.35 g, 1.51 x 10⁻³ moles) was heated to 180 °C under reduced pressure (0.02 mbar) until no further evolution of gas was observed. The resultant yellow glass was dissolved in methanol (50 ml) and sodium hydroxide (0.23 g, 1.51 x 10⁻³ moles) was added, the resultant mixture was brought to reflux. After refluxing overnight, a precipitate had formed which was collected via centrifugation and washed with methanol (3 x 50 ml). The white solid was dried under reduced pressure (0.02 mbar, 70 °C). Yield of 26 (0.52 g, 60 %).
¹H NMR (300 MHz, CDCl₃) 7.96-7.89 (m, 4 H), 7.62-7.46 (m, 6 H), 7.39 (tt, 1 H), 7.31-7.21 (m, 2 H), 7.10 (s, 1 H), 7.04 (s, 1 H), 7.01 (s, 1 H), 3.05 (m, 4 H), 1.89 (m, 4 H), 1.54-1.37 (m, 12 H), 0.95-0.89 (m, 6 H).
¹³C NMR (75 MHz, CDCl₃) 157.64, 157.31, 156.83, 155.20, 154.37, 154.24, 130.79, 130.04, 129.96, 129.76, 129.24, 129.11, 127.30, 127.10, 126.15, 126.09, 125.35, 124.17, 123.22, 122.20, 121.80, 121.03, 115.57, 115.33, 111.44, 102.08, 101.11, 100.64, 32.11, 32.10, 30.37, 30.34, 30.21*, 29.62*, 23.05*, 14.58, 14.55. (* indicates two overlapping signals) MALDI-MS 594 [M]⁺

Elemental Analysis C:80%, H:7.13%.

Quantum efficiency [cyclohexane measured relative to anthracene (0.26)] 0.7±0.2

### Example 3

### Preparation of Unsubstituted Benzofuran Trimer (Compound 36)

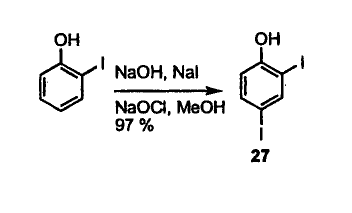

See *J. Org. Chem.* **1990,** *55*, 5287-5291. Yield 77 %.

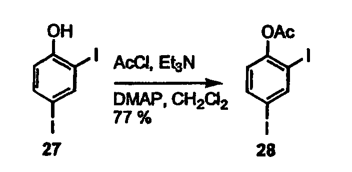

See *Tetrahedron* **1995,** *51,* 8199-8212. Yield 97 %.

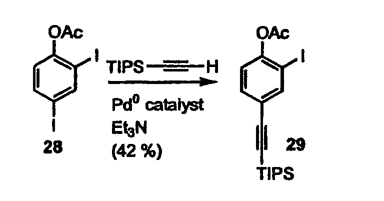

Modification of the method outlined by R. W. Bates, C. J. Gabel, J. Ji, T. Rama-Devi, *Tetrahedron,* **1995,** *51,* 8199-8212.

Aryl iodide **28** (10 g, 2.58 x 10⁻² moles), palladium(II) acetate (116 mg, 5.2 x 10⁻⁴ moles), copper(I) iodide (50 mg, 2.6 x 10⁻⁴), and triphenylphosphine (262 mg, 1.0 x 10⁻⁴ moles) were dissolved in triethylamine (60 ml, freshly distilled ex. CaH₂), and the mixture degassed using two freeze thaw saturate with nitrogen cycles. Tri*iso*propylsilylacetylene (4.7 g, 5.8 ml, 2.6 x 10⁻² moles) was then added *via* syringe and the mixture degassed by boiling under reduced pressure and then flushing with nitrogen. After three days stirring at room temperature hexanes was added and the triethylamine hydrogen iodide removed *via* filtration through Celite. The filtrate was evaporated and then chromatographed on silica eluting with hexanes containing 0.25 % diethylether, to yield **29** as a white solid 4.8 g, 42 %.
¹H NMR (300 MHz, CDCl₃) 7.93 (d, J = 2 Hz, 1 H), 7.46 (dd, J = 2 and 8 Hz, 1 H), 7.03 (d, J = 8 Hz, 1 H), 2.36 (s, 3 H), 1.13 (s, 3 H).
¹³C NMR (75 MHz, CDCl₃) 168.75, 151.55, 142.88, 133.48, 123.65, 123.01, 104.77, 92.82, 90.52, 21.61, 19.06, 11.66.
GC EI-MS 442 [M]⁺, 399 [M - Ac]⁺

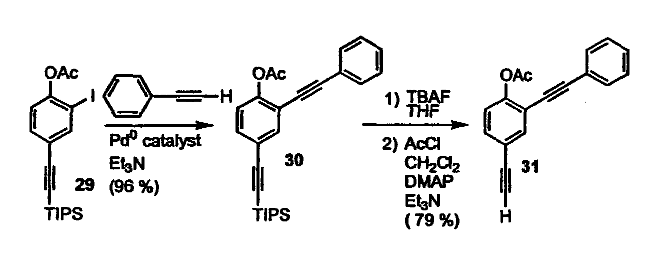

Aryl iodide **29** (2.4 g, 5 x 10⁻³ moles), palladium(II) acetate (24 mg, 1.1 x 10⁻⁴ moles), copper(I) iodide (10 mg, 5.4 x 10⁻⁵ moles), and triphenylphosphine (57 mg, 2.2 x 10⁻⁴ moles) were dissolved in triethylamine (20 ml, freshly distilled ex. CaH₂) and the resulting mixture degassed using two freeze thaw saturate with nitrogen cycles. Phenylacetylene (665 mg, 715 µl, 6.5 x 10⁻³ moles) was added *via* syringe and the resulting solution degassed by boiling under reduced pressure and saturating with nitrogen. The reaction mixture was heated to 70 °C for 6 hours. The reaction mixture was then filtered through Celite, the Celite was washed with hexanes and then the solvent evaporated. Chromatography was carried out on silica eluting with dichloromethane and hexanes (1:3). The resulting colourless oil 7 (2.16 g, 96 %), was then taken on to the next step without further purification or characterisation. To a solution of triisopropyl protected acetylene **30** (2.0 g, 4.8 x 10⁻³ moles) dissolved in dichloromethane tetrabutylammonium fluoride (1 M in THF, 4.8 ml, 4.8 x 10⁻³ moles) was added. The reaction was complete after 15 minutes stirring at room temperature. The reaction was quenched by the addition of calcium chloride and brine, the product was extracted with dichloromethane, the organic fractions were dried over magnesium sulphate and then the solvent evaporated. A re-acylation step was then carried out because some of the acetate protecting groups were lost during the TBAF deprotection reaction. To a mixture of crude **31,** dissolved in dry dichloromethane (50 ml), triethylamine (1.0 ml, .73 g, 7.2 x 10⁻³ moles) and DMAP (41 mg, 3.7 x 10⁻⁴ moles) was slowly added acetyl chloride (0.6 g, 0.5 ml, 6.4 x 10⁻³ moles). The resulting mixture was left to stir overnight and then quenched by washing with ammonium chloride solution (100 ml, 10 % solution) followed by sodium hydrogen carbonate (100 ml 5 % solution). The organic fractions were dried over magnesium sulphate and the solvent evaporated. Chromatography on silica eluting with dichloromethane : hexanes (1:1) yielded 0.99 g, 79 % of **31** as a cream coloured crystalline solid.
¹H NMR (300 MHz, CDCl₃) 7.71 (d, J = 2 Hz, 1 H), 7.52-7.44 (m, 3 H), 7.40-7.34 (m, 3 H), 7.10 (d, J = 8 Hz, 1 H), 3.09 (s, 1 H), 2.37 (s, 3 H).
¹³C NMR (75 MHz, CDCl₃) 169.02, 152.00, 136.97, 133.44, 132.03, 129.26, 128.88, 122.98, 120.66, 118.30, 95.28, 83.70, 82.43, 78.35, 21.27.
GC EI-MS 260 [M]⁺, 218 [M - Ac]⁺

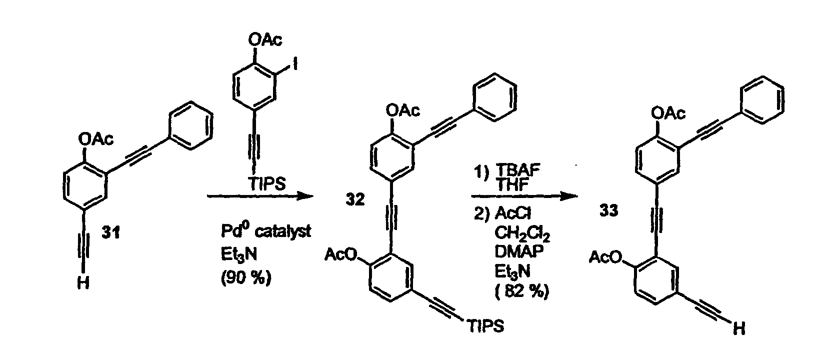

Aryl acetylene **31** (0.9 g, 4 x 10⁻³ moles), aryl iodide **29** (1.5 g, 3.5 x 10⁻³ moles), palladium(II) acetate (16 mg, 6.9 x 10⁻⁵ moles), copper(I) iodide (7 mg, 3.5 x 10⁻⁵ moles), and triphenylphosphine (36 mg, 1.4 x 10⁻⁴ moles) were dissolved in triethylamine (10 ml, freshly distilled ex. CaH₂) and the resulting mixture degassed using two freeze thaw saturate with nitrogen cycles. After heating to 70 °C for 6 hours the reaction mixture was filtered through Celite, the Celite was washed with hexanes and then the solvent evaporated. Chromatography was carried out on silica eluting with dichloromethane and hexanes (1:1). The resulting white solid **32** (1.79 g, 90 %), was then taken on to the next step without further purification or characterisation. To a solution of triisopropyl protected acetylene **32** (1.6 g, 2.8 x 10⁻³ moles) dissolved in dichloromethane (80 ml) tetrabutylammonium fluoride (1 M in THF, 2.8 ml, 2.8 x 10⁻³ moles) was added. The reaction was complete after 15 minutes stirring at room temperature. The reaction was quenched by the addition of calcium chloride and brine, the product was extracted with dichloromethane, the organic fractions were dried over magnesium sulphate and then the solvent evaporated. A reacylation step was then carried out because some of the acetate protecting groups were lost during the TBAF deprotection reaction. To a mixture of crude **31**, dissolved in dry dichloromethane (80 ml), triethylamine (1.3 ml, 0.93 g, 9.2 x 10⁻³ moles) and DMAP (51 mg, 4.5 x 10⁻⁴ moles) was slowly added acetylchloride (0.72 g, 0.66 ml, 9.2 x 10⁻³ moles). The resulting mixture was left to stir overnight and then quenched by washing with ammonium chloride solution (60 ml, 10 % solution) followed by sodium hydrogen carbonate (60 ml 5 % solution). The organic fractions were dried over magnesium sulphate and the solvent evaporated. Chromatography on silica eluting with dichloromethane : hexanes (1:1) yielded 0.94 g, 81 % of **31** as a cream coloured crystalline solid.
¹H NMR (300 MHz, CDCl₃) 7.70-7.68 (m, 2 H), 7.53-7.43 (m, 4 H), 7.40-7.34 (m, 3 H), 7.13 (d, J = 9 Hz, 1 H), 7.11 (d, J = 9 Hz, 1 H), 3.09 (s, 1 H), 2.384 (s, 3 H), 2.378 (s, 3 H).
¹³C NMR (75 MHz, CDCl₃) 169.05, 168.98, 152.01*, 137.05, 136.34, 133.70, 132.90, 132.03, 129.29, 128.89, 123.13, 123.03, 122.94, 121.12, 120.70, 118.47, 117.90, 95.41, 93.49, 84.29, 83.68, 82.68, 82.33, 78.41, 21.32, 21.29. (* indicates two overlapping signals)
GC EI-MS 418 [M]⁺, 376 [M - Ac]⁺, 334 [M - 2Ac]⁺

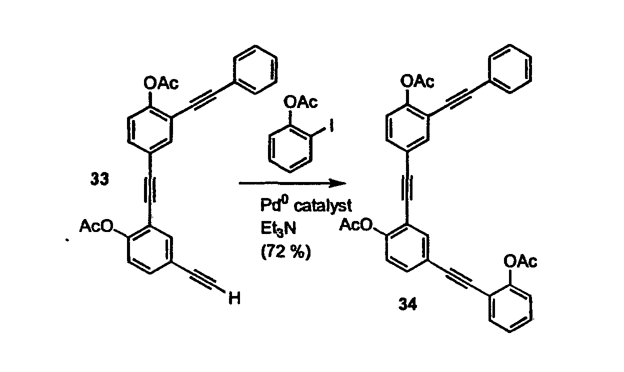

Aryl acetylene **33** (0.9 g, 2 x 10⁻³ moles), aryl iodide **37** (0.53 g, 0.31 ml, 2 x 10⁻³ moles), palladium(II) acetate (20 mg, 9 x 10⁻⁵ moles), copper(I) iodide (9 mg, 4.5 x 10⁻⁵ moles), and triphenylphosphine (47 mg, 1.8 x 10⁻⁴ moles) were dissolved in triethylamine (12 ml, freshly distilled ex. CaH₂) and the resulting mixture degassed using two freeze thaw saturate with nitrogen cycles. After heating to 70 °C for 6 hours the reaction mixture was filtered through Celite, the Celite was washed with hexanes and then the solvent evaporated. Chromatography was carried out on silica eluting with dichloromethane and hexanes (3:1). The resulting white solid **34** was recrystallised from chloroform with layered addition of hexanes to yield a white solid (0.98 g, 87 %).
¹H NMR (300 MHz, CDCl₃) 7.72-7.68 (m, 2 H), 7.59-7.55 (m, 1 H), 7.53-7.45 (m, 4 H), 7.42-7.35 (m, 4 H), 7.28-7.22 (m, 1 H), 7.16-7.12 (m, 3 H), 2.38 (as, 9 H).
¹³C NMR (75 MHz, CDCl₃) 169.33, 169.06*, 152.01, 151.97, 151.84, 136.36, 133.51, 133.14, 132.92, 132.04, 130.04, 130.21, 129.30, 128.89, 126.42, 123.14, 122.94, 122.77, 121.52, 121.13, 118.48, 117.99, 117.40, 95.42, 93.53, 92.74, 85.36, 84.36, 83.67, 21.37, 21.34, 21.30. (* indicates two overlapping signals)
APCI-MS 553 [M]⁺, 695 [M - Ac]⁺, 595 [M - 2Ac]⁺

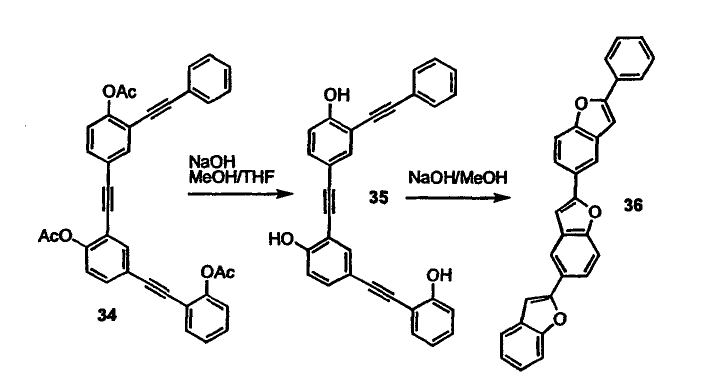

Triacetate **34** (0.9 g, 1.6 x 10⁻³ moles) was dissolved in tetrahydrofuran (45 ml). To this solution, methanolic sodium hydroxide (0.2 g, 5 x 10⁻³ moles, dissolved in 4.5 ml methanol) was added. The resultant mixture was then left to stir overnight. The reaction mixture was then neutralised with dilute hydrochloric acid and then poured into ether. The ether layer was washed with water, dried over magnesium sulphate and then evaporated. The resultant solid was recrystallised from dichloromethane with layered addition of hexanes to yield **35** (650 mg, 94 %). The triphenol **35** was then converted to the benzofuran trimer **36** without further characterisation or purification. The triphenol **35** (500 mg, 1.2 x 10⁻³ moles) and sodium hydroxide (0.14 g, 3.5 x 10⁻³ moles) were dissolved in methanol (100 ml). The mixture was degassed and then left to reflux overnight. The resultant white precipitate was collected *via* centrifugation to yield **36** (470 mg, 94 %) as a white crystalline solid.
¹H NMR (300 MHz, CDCl₃) 8.13 (d, J = 1.4 Hz, 1 H), 8.12 (d, J = 1.7 Hz, 1 H), 7.92-7.85 (m, 2 H), 7.85-7.75 (m, 2 H), 7.62-7.31 (m, 8 H), 7.32-7.20 (m, 1 H), 7.10 (s, 1 H), 7.07 (s, 1 H), 7.03 (s, 1 H).
Material not soluble enough for ¹³C NMR.
EI-MS 426 [M]⁺
Elemental Analysis C:84.57%, H:4.31%.
Quantum efficiency [cyclohexane measured relative to anthracene (0.26)] 0.7±0.2

### Example 4

### Benzofuran Trimer with a Different Geometry

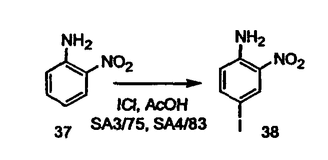

See *Bull*. *Soc*. *Chim*. *France,* **1902,** 964.

Iodine monochloride (25 g, 0.15 moles) in acetic acid (10 ml) was added dropwise to o-nitroaniline **37** (21.3 g, 0.15 moles) in acetic acid (20 ml). A precipitate formed after 4 h. ¹H NMR of the crude reaction mixture showed 70 % of the iodinated starting material and 30 % unreacted starting material. Steam distillation was carried out to remove the excess starting material and the residue was allowed to cool before collecting the solid by filtration. Recrystallisation from ethanol yielded 26 g, 64 % of 2-nitro-4-iodoaniline **38**.

¹H NMR (300 MHz, CDCl₃) 8.43 (d, J = 2 Hz, 1 H), 7.57 (dd, J = 2, 9 Hz, 1 H), 6.61 (d, J = 9 Hz, 1 H), 4.0 (brs, 2 H).

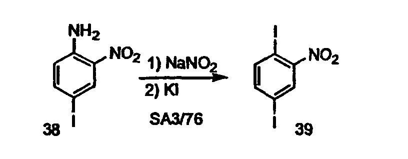

2-nitro-4-iodo aniline (26 g, 0.1 moles) was dissolved in acetic acid (70 ml), sulphuric acid (70 ml) and water (75 ml). The mixture was cooled to 0 °C and then an aqueous solution of sodium nitrite (7 g, 0.1 moles dissolved in ice cold water (30 ml)) added over 1 h. Potassium iodide (16.6. g, 0.1 moles dissolved in ice cold water (30 ml)) was then carefully added dropwise to the solution of the diazonium salt and the temperature of the resulting mixture slowly increased to 60 °C, on cooling this solution and addition of water (200 ml) a precipitate formed which was collected by filtration. The precipitate was dissolved in ethanol (350 ml) and activated charcoal (5 g) added, the activated charcoal was then removed by filtration leaving a yellow/orange solution, from which pale/orange crystals formed. The crystalline product was dried to yield 28 g, 75 % of **39**.
¹H NMR (300 MHz, CDCl₃) 8.14 (d, J = 2 Hz, 1 H), 7.73 (d, J = 8 Hz, 1 H), 7.56 (dd, J = 2, 8 Hz, 1 H).
¹³C NMR (75 MHz, CDCl₃) 153.82, 143.42, 142.75, 134.43, 93.26, 85.99.

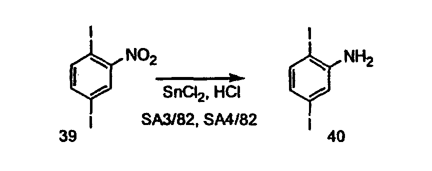

The nitrobenzene diiodide (30 g, 8 x 10⁻² moles) was added to hydrochloric acid (100 ml), and then tin(II)chloride dihydrate (55 g, 2.4 x 10⁻¹ moles) was added portionwise. The mixture was warmed to 50 °C for 2 h before testing by t1c. Since some starting material remained, a further portion of tin(II)chloride dihydrate (17 g, 7.3 x 10⁻² moles) was added and the mixture left stirring at 50 °C overnight. Neutralisation was carried out with sodium hydroxide, the precipitate that formed was collected by filtration, dried, and then dissolved in chloroform. Any chloroform insoluble material was removed by filtration, a pale yellow solid was formed when the chloroform was evaporated from the supernatant yielding (23 g, 84 %) of **40.**
¹H NMR (300 MHz, CDCl₃) 7.30 (d, J = 8 Hz, 1 H), 7.05 (d, J = 2 Hz, 1 H), 6.76 (dd, J = 2, 8 Hz, 1 H), 4.09 (br.s, 2 H).
¹³C NMR (75 MHz, CDCl₃) 148.47, 140.56, 129.22, 123.42, 94.95, 83.82.

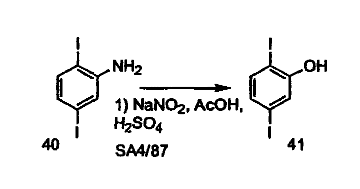

The aniline diiodide (23 g, 6.7 x 10⁻² moles) was dissolved in a tepid mixture of actetic and sulphuric acids. This mixture was cooled to 0 °C. Sodium nitrite (4.6 g, 6.7 x 10⁻² moles) was then added portionwise whilst maintaining the temperature below 5 °C. After stirring for 1 h, the mixture was poured slowly onto crushed ice (460 g), and then the temperature was slowly increased to 60 °C. The mixture was then diluted with water, and steam distilled to yield 11 g, 43 % of **41**.
¹H NMR (300 MHz, CDCl₃) 7.34 (d, J = 8 Hz, 1 H), 7.34 (d, J = 2 Hz, 1 H), 7.00 (dd, J = 2, 8 Hz, 1 H), 5.26 (br.s, 2 H).
¹³C NMR (75 MHz, CDCl₃) 155.86, 139.64, 131.29, 126.29, 124.62, 94.88, 85.76.

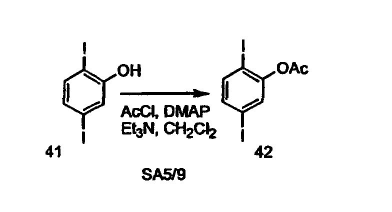

Acetylchloride (2.7 g, 2.5 ml, 3.5 x 10⁻² moles) was added dropwise to a solution of triethylamine (3.5 g, 4.8 ml, 3.5 x 10⁻² moles), **41** (10 g, 2.9 x 10⁻² moles) and dimethylaminopyridine (234 mg, 2.08 x 10⁻³moles) in dichloromethane (220 ml) at 0 °C. The mixture was stirred for 1 h, washed with aqueous ammonium chloride (500 ml, 10 % solution) and aqueous sodium bicarbonate (500 ml, 5 % solution). The organic layer was dried (MgSO₄) and evaporated. The crude product was chromatographed on silica eluting with hexane containing 5 % ethyl acetate and then recrystallised from a minimum volume of hot hexane to yield **42** (10.9 g, 97 %).
¹H NMR (300 MHz, CDCl₃) 7.52 (d, J = 8 Hz, 1 H), 7.43 (d, J = 2 Hz, 1 H), 7.30 (dd, J = 2, 8 Hz, 1 H).
¹³C NMR (75 MHz, CDCl₃) 168.66, 152.11, 140.87, 137.17, 132.50, 93.61, 90.76, 21.56.
GC EI-MS 388 [M]⁺, 346 [M - Ac]⁺

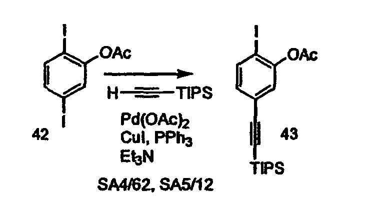

Using a modification of the method outlined by R. W. Bates, C. J. Gabel, J. Ji, T. Rama-Devi, (*Tetrahedron*, **1995,** *51,* 8199-8212), aryl iodide **42** (10 g, 2.58 x 10⁻² moles), palladium(II) acetate (116 mg, 5.2 x 10⁻⁴ moles), copper(I) iodide (50 mg, 2.6 x 10⁻⁴), and triphenylphosphine (272 mg, 1.0 x 10⁻⁴ moles) were dissolved in triethylamine (60 ml, freshly distilled ex. CaH₂), and the mixture degassed using two freeze thaw saturate with nitrogen cycles. Tri*iso*propylsilylacetylene (4.7 g, 5.8 ml, 2.6 x 10⁻² moles) was then added *via* syringe and the mixture degassed by boiling under reduced pressure and then flushing with nitrogen. After three days stirring at room temperature hexanes was added and the triethylamine hydrogen iodide removed *via* filtration through Celite. The filtrate was evaporated and then chromatographed on silica eluting with hexanes containing 2.5 % ethylacetate, to yield **43** as a white solid 5 g, 44 %.
¹H NMR (300 MHz, CDCl₃) 8.2 (d, J = 8 Hz, 1 H), 7.18 (d, J = 2 Hz, 1 H), 7.06 (dd, J = 8 and 2 Hz, 1 H), 2.36 (s, 3 H), 1.10 (s, 3 H).
GC EI-MS 442 [M]⁺, 399 [M - Ac]⁺

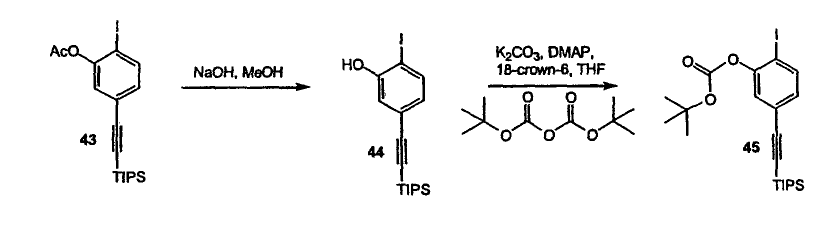

Sodium hydroxide (0.45 g, 1.1 x 10⁻² moles) was dissolved in methanol (2 ml) and added to tri*iso*propylsilyl protected acetylene **43** (5 g, 1.1 x 10⁻² moles) dissolved in tetrahydrofuran (75 ml). After stirring for 1 hour the base was neutralised with hydrochloric acid (5 % aqueous). The mixture was then thoroughly extracted with diethylether. The organic fractions were dried over magnesium sulphate, and then evaporated. The colourless oil was carefully dried under high vacuum (2 x 10⁻² mbar) to yield **44.** All of **44** was then taken on to the next step without further purification or characterisation. Phenol **44,** potassium carbonate (2.45. g, 1.8 x 10⁻² moles), dimethylaminopyridine (catalytic amount) and 18-crown-6 (catalytic amount) were dried under vacuum and then flushed with nitrogen. Tetrahydrofuran (65 ml, dry and oxygen free) was then added *via*syringe followed by BOC-anhydride (2.84 g, 1.3 x10⁻² moles). The reaction was then left to stir until no starting material was observed by tlc (1 hour). The reaction was quenched by the addition of brine and the resulting mixture extracted with diethylether. The organic fractions were then dried over magnesium sulphate and evaporated. The pale yellow oil was chromatographed on silica eluting with dichloromethane and hexanes (1:3) to give a waxy solid which was recystallised from pentane to yield 5.36 g, 95 % of **45.**
¹H NMR (300 MHz, CDCl₃) 7.75 (d, J = 8 Hz, 1 H), 7.26 (d, J= 2 Hz, 1 H), 7.07 (dd, J = 2, 8 Hz, 1 H), 1.59 (s, 9 H), 1.12 (s, 21 H).
¹³C NMR (75 MHz, CDCl₃) 151.35, 150.91, 139.41, 131.27, 126.16, 125.43, 105.146, 93.51, 91.17, 84.66, 27.96, 18.85, 11.46.

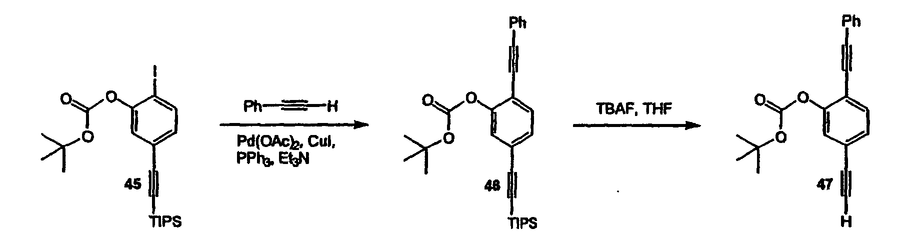

Aryl iodide **45** (2.5g, 5.0 x 10⁻³ moles), palladium(II) acetate (22.2 mg, 1.0 x 10⁻⁴ moles), copper(I) iodide (9.5 mg, 5.0 x 10⁻⁵ moles), and triphenylphosphine (53 mg, 2.0 x 10⁻⁴ moles) were dissolved in triethylamine (25 ml, freshly distilled ex. CaH₂) and the resulting mixture degassed using two freeze thaw saturate with nitrogen cycles. Phenylacetylene (572 mg, 615 µl, 5.6 x 10⁻³ moles) was added via syringe and the resulting solution degassed by boiling under reduced pressure and saturating with nitrogen. The reaction mixture was heated to 70 °C for 3 hours. The reaction mixture was then filtered through celite, the celite was washed with hexanes and then the solvent evaporated. Chromatography was carried out on silica eluting with dichloromethane and hexanes (1:3). The resulting waxy solid **46** (2.3 g, 93 %), was then taken on to the next step without further purification or characterisation. To a solution of tri*iso*propyl protected acetylene **46** (2.2 g, 4.7 x 10⁻³ moles) dissolved in dichloromethane (100 ml) tetrabutylammoniumfluoride (1 M in THF, 4.7 ml, 4.7 x 10⁻³ moles) was added. The reaction was complete after 10 minutes stirring at room temperature. The reaction was quenched by the addition of calcium chloride and brine, the product was extracted with dichloromethane, the organic fractions were dried over magnesium sulphate and then the solvent evaporated. Chromatography on silica eluting with dichloromethane : hexanes (2:1) followed by recrystallisation from pentane yielded 1.4 g, 94 % of **47** as a waxy pale yellow solid.
¹H NMR (300 MHz, CDCl₃) 7.56-7.49 (m, 3 H), 7.39-7.33 (m, 4 H), 7.32 (d, J = 2, 1 H), 3.20 (s, 1 H), 1.52 (s, 9 H).
¹³C NMR (75 MHz, CDCl₃) 151.52, 151.22, 133.00, 131.91, 129.90, 129.00, 128.56, 125.87, 123.44, 122.89, 118.69, 96.49, 84.30, 83.81, 82.52, 79.85, 27.84.

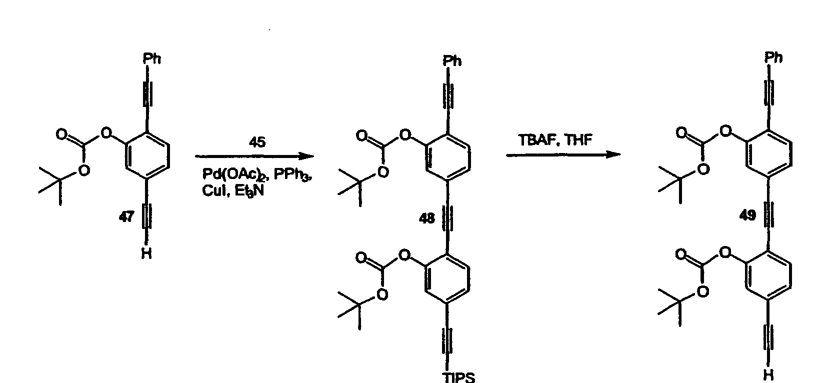

Aryliodide **47** (2.2 g, 4.4x 10⁻³ moles), arylacetylene **45** (1.4 g, 4.4 x 10⁻³ moles), palladium(II) acetate (19.1 mg, 9.0 x 10⁻⁵ moles), copper(I) iodide (8.3 mg, 4.3 x 10⁻⁵ moles) and triphenylphosphine (45 mg, 1.7 x 10⁻⁴ moles) were dissolved in triethylamine (25 ml, freshly distilled ex. CaH₂) and the resulting mixture degassed by two freeze thaw saturate with nitrogen cycles. The reaction mixture was stirred at 70 °C for 3 hours, by which time no starting materials were visible by tlc. The reaction mixture was filtered through celite, the celite being carefully washed with hexanes. The solvent was then evaporated and the resulting pale yellow oil chromatographed on silica eluting with a mixture of hexanes and dichloromethane (2:1). The resulting solid **48** was dissolved in dichloromethane (100 ml) and tetrabutylammonium fluoride (1 M in THF, 3.54 ml, 3.54 x 10⁻³ moles) added. The reaction was quenched by the addition of calcium chloride and brine, the product was extracted with dichloromethane, the organic fractions were dried over magnesium sulphate and then the solvent evaporated. Chromatography on silica eluting with a mixture of dichloromethane and hexanes (1:1) yielded **10** (1.29 g, 91 %) as waxy solid which was recystallised from pentane 1.9g, 80% .
¹H NMR (300 MHz, CDCl₃) 7.64-7.57 (m, 4 H), 7.41-7.32 (m, 7 H), 3.22 (s, 1 H), 1.53 (s, 18 H).
¹³C NMR (75 MHz, CDCl₃) 151.66*, 151.64, 151.196, 133.06, 133.00, 131.92, 129.95, 129.37, 129.03, 128.59, 125.95, 125.43, 123.95, 123.94, 122.92, 118.55, 118.14, 96.72, 95.11, 86.29, 84.50, 84.28, 83.95, 82.45, 80.14, 27.85*.

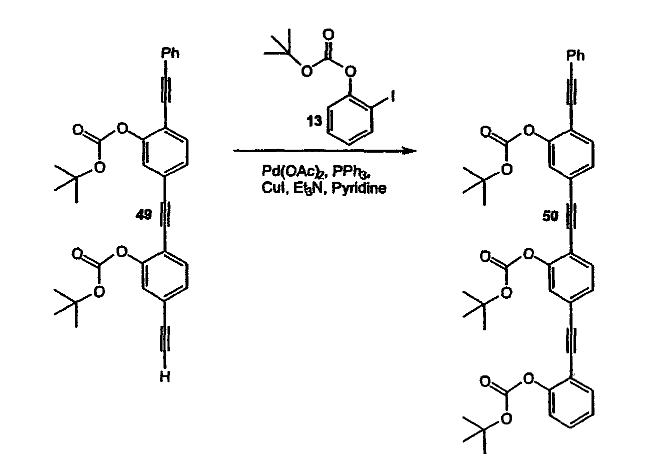

Aryl iodide **13** (1.25 g, 3.91 x 10⁻³ moles), arylacetylene **49** (1.9 g, 3.55 x 10⁻³ moles), palladium(II) acetate (17.8 mg, 8.0 x 10⁻⁵ moles), copper(I) iodide (7.6 mg, 4 x 10⁻⁵ moles) and triphenylphosphine (42 mg, 1.6 x 10⁻⁴ moles) were dissolved in a mixture of triethylamine (10 ml, freshly distilled ex CaH₂) and pyridine (10 ml, freshly distilled ex CaH₂) the resulting mixture degassed using two freeze thaw saturate with nitrogen cycles. The reaction mixture was stirred at 70 °C overnight. The reaction mixture was filtered through celite, the celite was carefully washed with hexanes and then the solvent evaporated to yield a yellow solid. This solid was chromatographed on silica eluting with a mixture of dichloromethane and hexanes (3:1) to give a mixture of the product and a significant impurity which could be removed by repeated recrystallisation from chloroform/hexane. The product was obtained as a bright yellow solid (750 mg, 30 %).
¹H NMR (300 MHz, CDCl₃) 7.60-7.50 (m, 5 H), 7.45-7.34 (m, 8 H), 7.24-7.18 (m, 2 H), 1.52 (s, 27 H).
¹³C NMR (75 MHz, CDCl₃) 151.99, 151.74, 151.63, 151.52, 151.17*, 133.20, 133.04, 132.97, 131.90, 130.22, 129.35*, 129.01, 128.57, 126.25, 125.42, 125.39, 124.80, 123.99, 122.89, 122.34, 118.49, 117.66, 117.27, 96.70, 95.13, 93.28, 87.04, 86.49, 84.40, 84.26, 84.14, 83.95, 27.86, 27.83*.

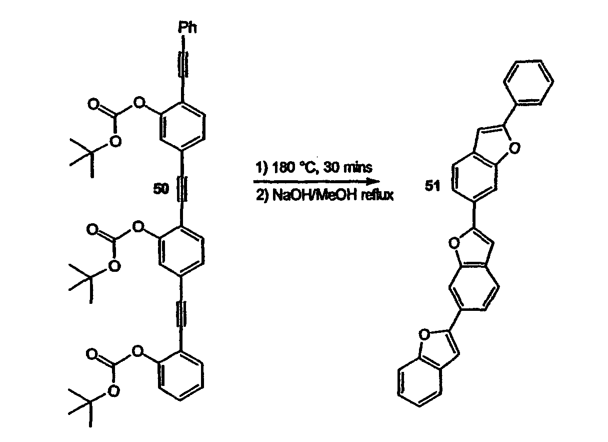

**50** (250 mg, 3.44 x 10⁻⁴ moles) was heated to 180 °C under reduced pressure (0.02 mbar) until no further evolution of gas was observed. The resultant yellow glass was dissolved in methanol (35 ml) and sodium hydroxide (0.042 g, 1.03 x 10⁻³ moles) was added, the resultant mixture was brought to reflux. After refluxing overnight a precipitate had formed which was collected *via* centrifugation and washed with methanol (3 x 50 ml). The white solid was sublimed (0.05 mbar, 250 °C). Yield of 5' (103 mg, 71 %)

¹H NMR (300 MHz, CDCl₃) 8.09-8.06 (m, 2 H), 7.93-7.91 (m, 2 H), 7.81-7.79 (m, 2 H), 7.68-7.40 (m, 8 H), 7.33-7.25 (m, 1H), 7.10 (s, 1 H), 7.09(m, 2H).

Alternatively, compound 43 containing appropriate substitutions can be used in the place of compound 4 and compound 17 described above.

### Example 5

### Preparation of Benzofuran Oligomers using Propylaminomethylated Polystyrene Supported Reactions.

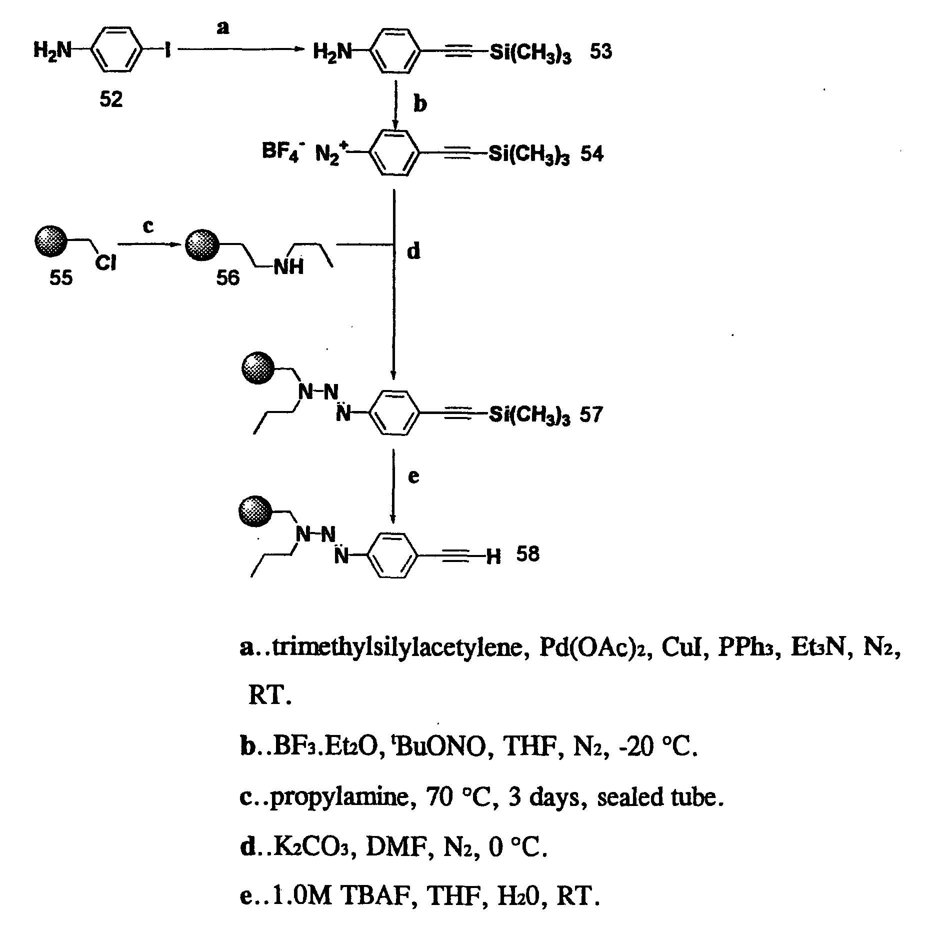

### Route to 2-Ethynyl-3-propyl-3-(benzylsupported)triazene Resin Precursor (50)

**1-(Trimethylsilylethynyl)-4-aminobenzene (53)** 4-Iodoaniline **(52)** (10.01 g, 45.7 mmol), palladium(II) acetate (210 mg, 9.3 mmol), copper(I) iodide (90 mg, 0.4 mmol), triphenylphosphine (480 mg, 1.8 mmol) and dry triethylamine (100 ml) were degassed under vacuum using two freeze-thaw-saturate with nitrogen cycles. Triemethylsilylacetylene (4.9 g, ca., 7.1 ml, 49.9 mmol) was then added using a syringe and septum, the reaction mixture was then very briefly degassed (caution as triemethylsilylacetylene is volatile!) before being purged with nitrogen and left to stir at room temperature for 18 h. The reaction mixture was then diluted with hexanes (150 ml) and the mixture filtered throught a pad of Hyflosupercel, the filtrate was then evaporated at reduced pressure to give a dark brown solid. Purified by flash chromatography [fine mesh silica gel; 1:1 dichloromethane-hexanes] to give an orange solid on evaporation. Recrystallised (hexanes) and dried in vacuo (1.0 mbar, 30 °C, 6 h). Yield = 7.22 g (83%). ¹H NMR (300 MHz; CDCl₃, TMS) d: 0.00 (9H, s, (CH₃)₃Si), 3.56 (2H, s broad, NH₂), 6.35 (2H, d, Ar-H_{3/5}, ³J_{HH} 8.54 Hz), 7.05 (2H, d, Ar-H_{2/6}, ³J_{HH} 8.13 Hz). ¹³C NMR(75 MHz, CDCl₃, TMS) δ: 0.00 ((CH₃)₃Si, no DEPT), 91.23 (Ar-CC-SiMe₃, no DEPT), 105.83 (Ar-CC-SiMe₃, no DEPT), 112.31 (Ar-C₁, no DEPT), 114.38 (Ar-C_{3/5}, +ve DEPT), 133.23 (Ar-C_{2/6}, +ve DEPT), 146.63 (Ar-C₄, no DEPT). FT-IR (KBr/DRIFT) υₘₐₓ: 3470, 3373, 2966, 2899, 2155, 1622, 1510, 1294, 1250, 844, 761, 698, 539 cm⁻¹.

**1-(Trimethylsilylethynyl)-4-diazoniumbenzene tetrafluoroborate (54)** Compound **53** (13.08 g, 69.2 mmol) in dry tetrahydrofuran (200 ml) was added dropwise at -20 °C over a period of 5 min to boron trifluoride diethyl etherate (35.1 ml, 39.39 g, 277 mmol) with constant stirring under a stream of dry nitrogen. Once the addition was complete tert-butyl nitrite (32.1 ml, 27.83 g, 269.9 mmol) in dry tetrahydrofuran (100 ml) was then added at -20 °C over a period of 30 min. After a further 10 mins stirring at -20 °C the mixture was then allowed to warm to 5 °C over 20 mins. Diethyl ether (400ml) was then added and the mixture chilled (ice-water bath) for 15 mins, the resulting crystalline solid was removed by filtration. The solid was washed with a minimum volume of cold diethyl ether and then dried in a vacuum. Yield = 15.26 g (77%). ¹H NMR (300 MHz; CDCl₃, TMS) δ: 0.28 (9H, s, (CH₃)₃Si), 7.72 (2H, d, Ar-H_{2/6}, ³JHH 9.0 Hz), 8.52 (2H, d, Ar-H_{3/5}, ³JHH 8.9 Hz). ¹³C NMR(75 MHz, CDCl₃, TMS) δ: -0.57 ((CH₃)₃Si, +ve DEPT), 101.69 (Ar-CC-SiMe₃, no DEPT), 109.36 (Ar-CC-SiMe₃, no DEPT), 112.47 (Ar-C₁, no DEPT), 132.70 (Ar-C₃, +ve DEPT), 134.12 (Ar-C₂, +ve DEPT), 136.57 (Ar-C₄, no DEPT). FT-IR (KBr/DRIFT) υₘₐₓ: 3105, 2959, 2291, 1578, 1252, 1070, 871, 845, 845, 763, 536 cm⁻¹.

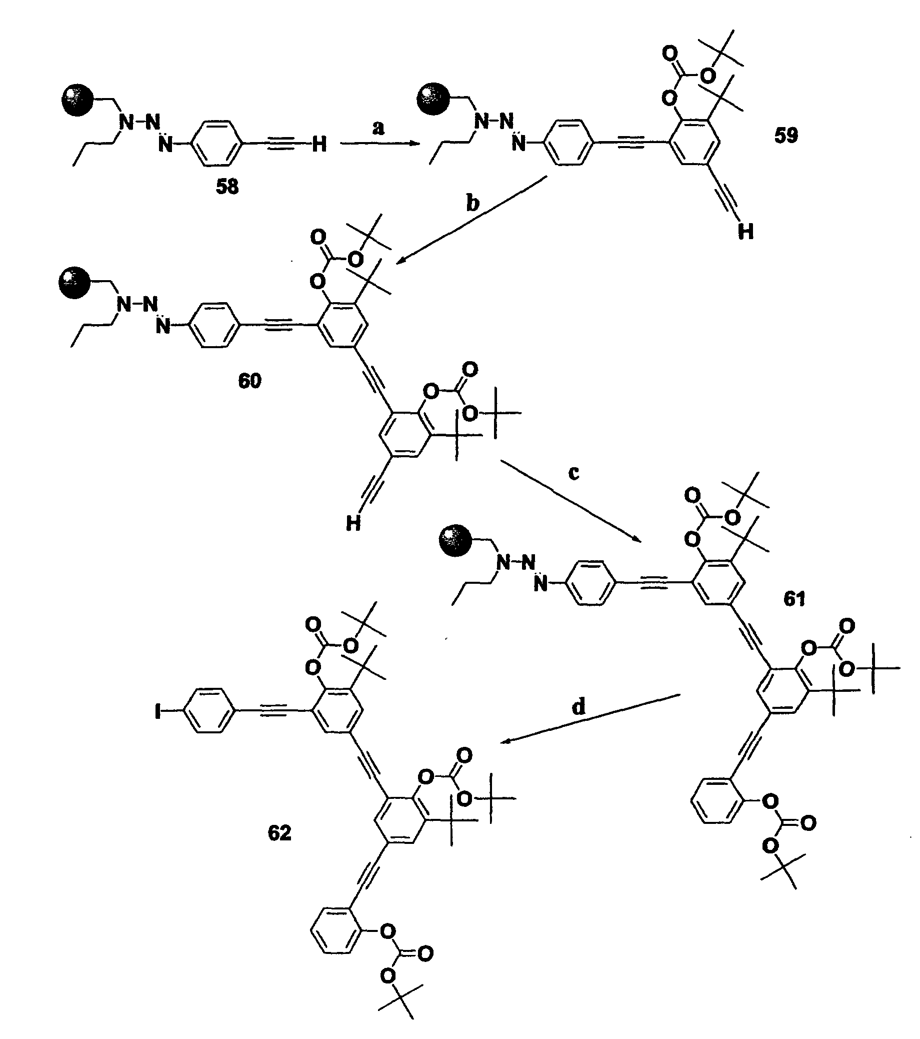

**Propylaminomethyl polystyrene (56)** Propylamine (30 ml) is placed in a thick walled tube and a stream of dry nitrogen gas is bubbled through it for 10 min. Merrifield's resin (chloromethyl polystyrene with 1 % divinylbenzene **(55))** (6.00 g) was then added portion wise ensuring careful mixing. The tube was then sealed and heated at 70 °C for three days with periodic agitation of the resultant polymer gel. After allowing to cool the polymer resin was transferred to a coarse sinter and washed thoroughly using dichloromethane (500 ml). Once dry the polymer resin was suspended in 1,4-dioxane/ 2N sodium hydroxide (1:1, 500 ml) and stirred at 70 °C for 30 min before being filtered through a sinter. This procedure was repeated for dioxane (500 ml), dioxane-water (1:1, 500 ml), dimethylformamide (500 ml), methanol (500 ml), and benzene (500 ml). The resulting polymer resin was then rinsed using hot methanol (500 ml), hot dichloromethane (500 ml) and methanol (250 ml) and then dried to constant mass in a vacuum. This was actually performed a further three times giving a total mass of 19.92 g. FT-IR (KBr/ DRIFT) vmax: 3058, 3026, 2918, 2849, 1601, 1493, 1452, 1030, 750, 541 cm-1. Elemental analysis: C 86.77; H 8.05; N 1.51. This corresponds to a degree of substitution (amine groups) of 1.075 m equivalents / g of polymer resin (as described by J.S. Moore *et al*, *J*. *Org*. *Chem*., 1996, **61,** 8163).

**2-(Trimethylsilyl)ethynyl-3-propyl-3-(benzylsupported)triazine Resin (57)** Compound **54** (4.92 g, 17.1 mmol),was added portionwise at 0 °C to a stirred slurry of polymer resin **56** (15.00 g), potassium carbonate (2.22 g, 16.1 mmol) in anhydrous dimethylformamide (240 ml) under a stream of dry nitrogen. After the addition of each portion an aliquot of of supernatant DMF solution was removed and mixed with triethylamine. This was then analysed by TLC to detect the presence of diethyl triazine (formed on completion of reaction and addition of **54** then stopped). The resin was then decanted into a sinter and washed with dimethylformamide (500 ml), water (500 ml), methanol (500 ml), tetrahydrofuran (500 ml) and methanol (500 ml). The resin was then dried in a vacuum. Yield = 17.30 g. FT-IR (KBr/DRIFT) υₘₐₓ: 3059, 3028, 2925, 2860, 2157 (Ar-CC-Rstr), 1600, 1493, 1453, 1429, 1249, 1090, 1030, 865, 846, 763, 704, 552 cm⁻¹. Elemental analysis: C 83.03; H 8.45; N 3.00. Corresponding to a degree of substitution (triazine groups) of 0.7155 m equivalents/ g of polymer resin.

**2-Ethynyl-3-propyl-3-(benzylsupported)triazine Resin (58)** Polymer resin **57** (16.01 g) was covered with tetrahydrofuran (180 ml) and tetrabutylammonium fluoride (15 ml, 1.0 M in THF/water) added by syringe with stirring at room temperature. The mixture turned a dark brown and was stirred for a further 30 mins, before being transferred to a sinter and washed carefully using tetrahydrofuran (600 ml) and methanol (600 ml). The resin was then dried to a constant mass in a vacuum. Yield = 14.30 g. FT-IR (KBr/DRIFT) υₘₐₓ: 3291 (CCHstr), 3059, 3027, 2926, 2920, 2853, 1601, 1494, 1453, 1189, 1103, 1036, 843, 762, 705, 543 cm⁻¹.

**Polymer Supported Diyne (59)** *Tris*(dibenzylideneacetone)dipalladium(0) (57.47 mg, 0.06 mmol), copper(I) iodide (22.86 mg, 0.12 mmol), triphenylphosphine (127.74 mg, 0.06 mmol) were dissolved in dry triethylamine (10 ml) in a Schlenk tube equipped with a magnetic stir bar. The mixture was degassed using two freeze-thaw-saturate with nitrogen cycles and then heated at 70 °C for 2 hours with stirring and under a stream of dry nitrogen. Once cool the supernatant liquid was removed by syringe (ca., 8 ml) and transferred to another Schlenk tube containing compound 58 (1.00 g), aryl iodide **6** (0.46g, 0.83 mmol). The reaction mixture was briefly degassed under vacuum and purged with dry nitrogen and heated at 70 °C for 24 h. Once cool the polymer resin was transferred to a sinter and washed sucessively with dichloromethane (2 x 30 ml), dimethylformamide (30 ml), 0.05 M sodium diethyldithiocarbamate /dimethylformamide (99:1, 30 ml), dimethylformamide (30 ml), dichloromethane (30 ml) and methanol (30 ml). The polymer resin was then dried in a vacuum. Yield = 1.08 g. FT-IR (powder/Si-C/DRFT) υₘₐₓ: 3086, 3065, 3039, 2931, 2922, 2866, 2156 (-CC-str), 1766 (C=Ostr), 1600, 1493, 1452, 1261, 1150, 897, 763, 748, 703, 539 cm⁻¹.

The polymer resin (1.05 g) was covered with dry tetrahydrofuran (12 ml) and tetrabutylammonium fluoride (0.8 ml, 1.0 M in THF/water solution, 0.8 mmol) added *via* syringe and the resulting slurry stirred at RT for 30 mins. The polymer resin was then transferred to a sinter and washed thoroughly with tetrahydrofuran (3 x 30 ml) and methanol (3 x 30 ml) before being dried in a vacuum. Yield = 0.90 g. FT-IR (powder/Si-C/DRIFT) υₘₐₓ: 3293 (Ar-CCHstr), 3083, 3061, 3025, 2962, 2947, 2933, 2922, 2850, 2210 (Ar-CC-Ar str), 1765 (C=Ostr), 1601, 1493, 1452, 1397, 1369, 1253, 1147, 1118, 1023, 857, 761, 703, 540 cm⁻¹.

**Polymer Supported Triyne (60)** A similar procedure to that previously described for compound **59** was used. Quantities used:- *tris*(benzylideneacetone)palladium(0) (56.21 mg, 0.06 mmol), copper(I) iodide (21.61 mg, 0.11 mmol), triphenylphosphine (127.41 mg, 0.49 mmol), dry triethylamine (10 ml), compound **59** (0.89 g) and aryl iodide **6** (0.46 g, 0.83 mmol). Yield = 1.01 g. FT-IR (powder/Si-C/DRIFT) υₘₐₓ: 3072, 3025, 2926, 2866, 2216 (A-CC-Arst), 2156 (Ar-CC-TIPS str), 1769 (C=Ostr), 1680 (C=Ostr), 1607, 1494, 1452, 1375, 1255, 1150, 884, 769, 704, 546 cm⁻¹. TBAF Deprotection:- polymer resin (1.00g), tetrabutylammonium fluoride (0.8 ml, 1.0 M THF/water solution, 0.8 mmol) and tetrahydrofuran (12 ml). Yield = 0.85 g. FT-IR (powder/Si-C/DRIFT) υₘₐₓ: 3291(Ar-CC-Hstr), 3059, 3025, 2972, 2926, 2217 (Ar-CC-Ar str), 1765 (C=Ostr), 1620, 1494, 1453, 1370, 1262, 1156, 889, 843, 764, 703, 540 cm⁻¹.

**Terminated Oligomer Resin (61)** A similar procedure to that previously described for compound **51** was used. Quantities used:- *tris*(benzylideneacetone)palladium(0) (60.5 mg, 0.07 mmol), copper(I) iodide (21.70 mg, 0.11 mmol), triphenylphosphine (133 mg, 0.51 mmol), dry triethylamine (10 ml), compound 52 (0.84 g), 1-(tertbutoxycarbonyloxy)-2-iodobenzene **13** (0.30 g, 0.93 mmol). Yield = 0.81 g. FT-IR (powder/Si-C/DRIFT) υₘₐₓ: 3065, 3032, 2927, 2918, 2216 (Ar-CC-Ar str), 1762 (C=O str), 1703 (C=O str), 1688 (C=O str), 1605, 1493, 1455, 1437, 1395, 1369, 1248, 1173, 1152, 890, 769, 702, 566 cm⁻¹.

**Triyne Trimer (62)** Compound **61** (0.79 g) and methyliodide (11 ml, 25.08 g, 176.7 mmol) were placed in a thick walled tube and degassed carefully using a stream of dry nitrogen for approximately 10 min. The tube was then sealed and heated at 110 °C for 12 h. The cooled reaction mixture was transferred to a flask using a small volume of chloroform and evaporated to give a brown solid. This solid was placed in a sinter and washed with hot chloroform (10 x 20 ml and the washings evaporated to give a brown oil. The oil was then purified by flash chromatography [fine mesh silica gel: 6:4 hexanes-dichloromethane (initially) and dichloromethane (finally) to give a yellow oil which was dried in a vacuum. Yield = 10 mg. ¹H NMR (300 MHz, CDCl₃, TMS) δ: 1.38, 1.39 (18H, s, (CH₃)₃CAr), 1.46, 1.48 (18H, s, (CH₃)₃CO), 1.54 (9H, s, (CH₃)₃COCO₂Ar₄), 7.20 (1H, d, Ar₄-H₃), 7.24 (1H, d, Ar-H₅), 7.25 (2H, d, Ar₁-H_{2/6}, 7.38 (1H, t, Ar₄-H₄), 7.52 (2H, d, Ar_{2&3}-H₄ and H₄), 7.57 (1H,dd, Ar₄-H₆), 7.61 (2H, dd, Ar_{2&3}-H₆ and H₆), 7.70 (2H, d, Ar₁-H_{3/5}). ¹³C NMR (75MHz; CDCl₃, TMS) δ: 28.06 ((CH₃)₃CO, +ve DEPT), 30.53 ((CH₃)₃CAr, +ve DEPT), 35.28 ((CH₃)₃CAr, no DEPT), 84.19, 84.26, 84.28 (Ar-CC-Ar, no DEPT), 84.53, 84.61 ((CH₃)₃CO, no DEPT), 85.82 ((CH₃)₃COCO₂Ar₄, no DEPT), 93.83, 93.88, 93.95 (Ar-CC-Ar, no DEPT), 95.05 (Ar₁-C₄-I, no DEPT), 117.75 (Ar₄-C₁, no DEPT), 119.54, 119.58 (Ar_{2/3}-C₁, no DEPT), 120.80, 120.93 (Ar_{2/3}-C₃, no DEPT), 122.48 (Ar₄-C₅, +ve DEPT), 122.72 (Ar₁-C₁, no DEPT), 126.40 (Ar₄-C₃, +ve DEPT), 130.07 (Ar₄- C₄, +ve DEPT), 131.32, 131.46 (Ar2/3C_{4/6}, +ve DEPT), 133.39, 133.49 (Ar₄-C_{3/5}, +ve DEPT), 134.37, 134.42 (Ar_{2/3}-C_{4/6}, +ve DEPT), 137.93 (Ar₁-C_{3/5}, +ve DEPT), 142.90, 142.96 (Ar_{2/3}-C_{6/5}, no DEPT), 150.85, 150.92 (Ar_{2/3}-C₆, no DEPT), 151.04 (OCO₂^{t}Bu, no DEPT), 151.74 (OCO₂^{t}Bu ₜₑᵣₘᵢₙₐₗ, no DEPT), 152.10 (Ar₄-C₆, no DEPT). FT-IR (powder/Si-C/DRIFT) υₘₐₓ:2992, 2880, 2256, 2210, 1769, 1494, 1370, 1282, 1226, 1156, 890, 820 cm⁻¹.

Removal of the tBOC groups, benzofuran formation and removal of the terminal iodide results in compound **12.**

### Example 6

### Preparation of a Phenylene Ethynylene Polymer and Conversion to a Benzofuran Polymer.

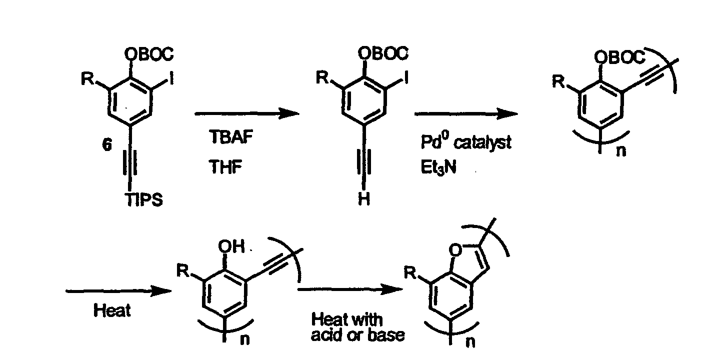

R = linear or branched alkyl, alkoxy etc,
For example R = tBu.

Compound **6** (0.0.81 g, 1.45x10⁻³ moles) was dissolved in dichloromethane (50 ml) and the mixture degassed by boiling under reduced pressure and then saturating with nitrogen. TBAF (1.6 ml, 1M solution in THF, 1.6x10⁻³ moles) was then added and the mixture left to stir for 15 mins. Work up was accomplished by the addition of calcium chloride followed by brine. The resultant mixture was extracted with dichloromethane. Chromatography on silica, eluting with a mixture of dichloromethane and hexane (1:3) yielded 0.53 g, 91 % of alkyne deprotected material as a white solid.
¹H NMR (300 MHz, CDCl₃) 7.84 (d, J=1.8 Hz, 1 H), 7.49 (d, J=2.0 Hz, 1 H), 3.07 (s, 1 H), 1.57 (s, 9 H), 1.33 (s, 9 H).
CI-MS 388 [M+NH₃]⁺, 318 [M+NH₃ - tBOC]⁺

The alkyne deprotected materials may then be polymerised in the presence of a Pd⁰ catalyst to produce precursor phenylene ethynylene polymers. The benzofuran polymers may be prepared by removal of the tBOC groups and cyclisation of the phenol onto the alkyne in an analogous manner to the preparation of **26**.

The advantage with the thermal removal of the tBOC groups is that it allows tBOC protected materials, eg compounds **11, 24** and precursor phenylene ethylene polymers, to be deposited to form thin films when they are very soluble in organic solvents and then to be converted thermally to form the required benzofuran oligomers etc via the corresponding phenols. Thus, it is possible to prepare the much less soluble benzofuran from a very soluble precursor. For example, the precursor phenylene ethylene polymers are soluble in organic solvents and forms thin films when spin-coated.

Thus, it is within the scope of the present invention to provide benzofuran, benzothiophene or indole polymers and co-polymers in addition to benzofuran, benzothiophene or indole oligomers and co-oligomers.

Embodiments of the present invention will now be described, by way of example, with reference to the accompanying drawings, in which:-
Fig. 1 shows EL spectra for the three benzofuran trimers, namely compounds **12**, **26** and **36**, in an electroluminescent device comprising, in sequence, an ITO electrode, a 60 nm NPB hole transport layer, a 60 nm electron transport layer containing the benzofuran trimer, and a Li:Al electrode,
Fig. 2 is a graph showing the IV characteristics for the device structures used to generate the data of Fig. 1,
Fig. 3 is a graph giving the IVL characteristics for a device structure comprising, in sequence, an ITO electrode, a 60 nm hole transport layer including compound **26,** a 60 nm electron transport layer containing tris-8-hydroxyquinolinealuminum, and a Li:Al electrode,
Fig. 4 is a graph showing the EL spectrum for compound **26** in the same device used to generate the data for Fig. 3,
Fig. 5 is a graph giving the IVL characteristics for a device comprising, in sequence, an ITO electrode layer, a 60 nm hole transporting layer containing NPB, a 30 nm active layer containing compound **26,** a 30 nm electron transport layer containing tris-8-hydroxyquinolinealuminum (Alq₃), and a Li:Al electrode,
Fig. 6 is a graph showing the EL spectrum for the device used to generate the IVL data of Fig. 5.

The data obtained in the above graphs was as a result of measurements carried out under a nitrogen atmosphere.

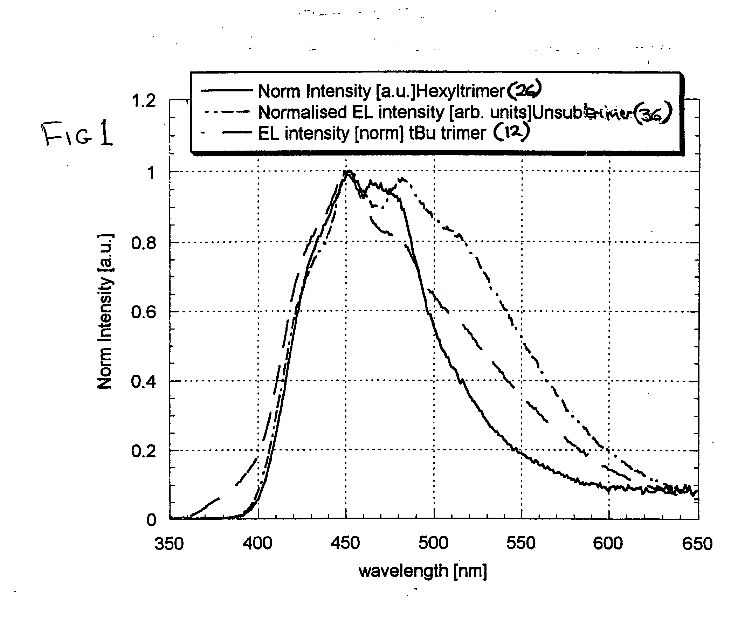

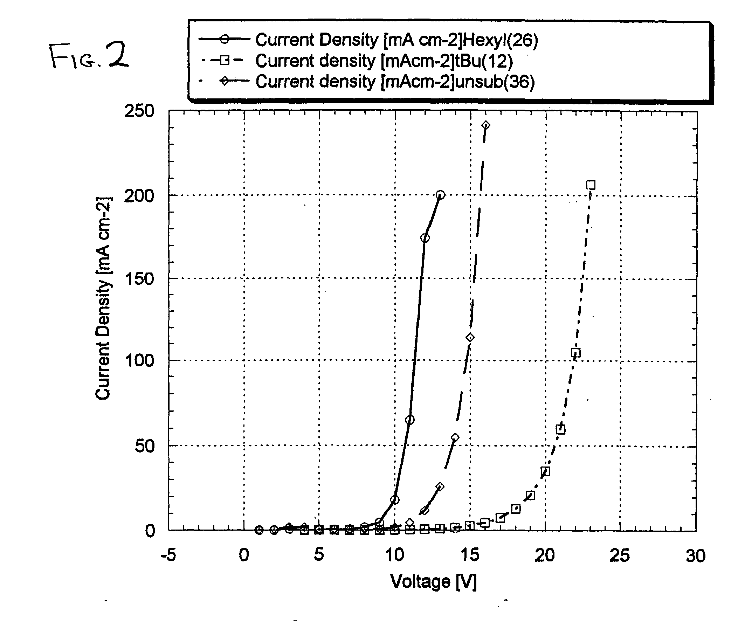

Referring now to Figs. 1 and 2, the light output of the device including compound **36** was 10 cdm⁻² at 16V and 240 mAcm⁻². The device including compound **12** produced a light output of 12 cdm⁻² at 23V and 206 mAcm⁻². The device using compound **26** had a light output of 1 cdm⁻² at 15V and 200 mAcm⁻². In all three cases, light emission appeared to be from the benzofuran trimer. The broadness of the emission spectrum is dependent upon the substituent in the trimer, indicating that there is scope for optimising the properties of the material by appropriate choice of the nature of the substituent group and the number of substituents.

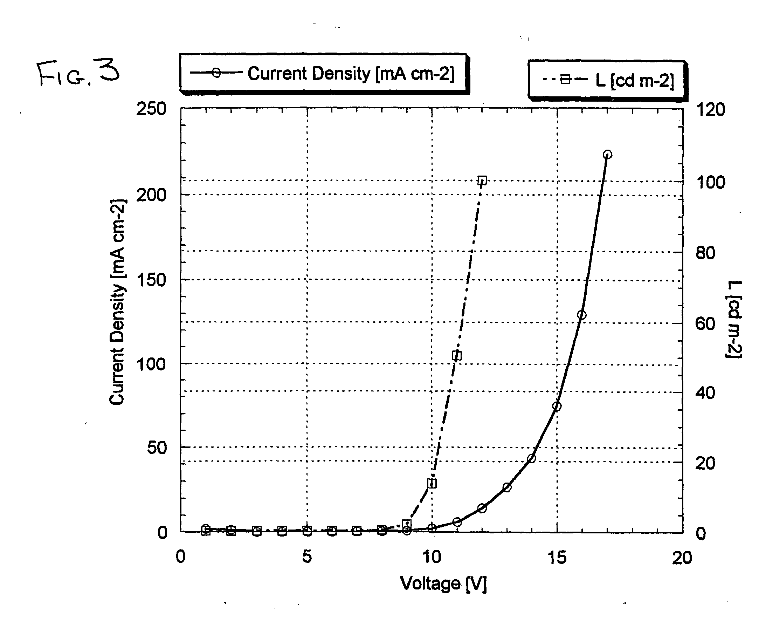

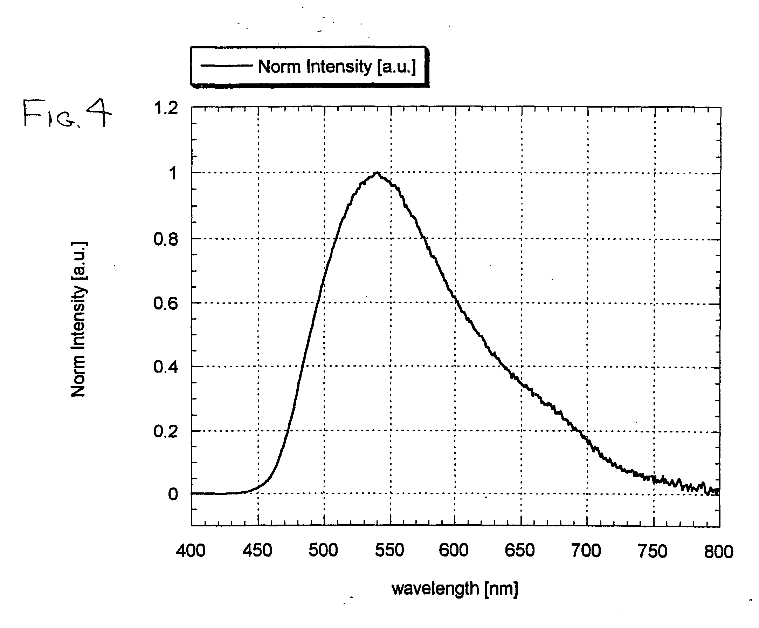

Referring now to Figs. 3 and 4, the benzofuran trimer, compound **26,** is used as a hole transport material and a light output of 100 cdm⁻² at 17V and 220 mAcm⁻². The CIE co-ordinates were x=0.36 and y=0.53. In this case, the emission came from the Alq₃ electron transport layer.

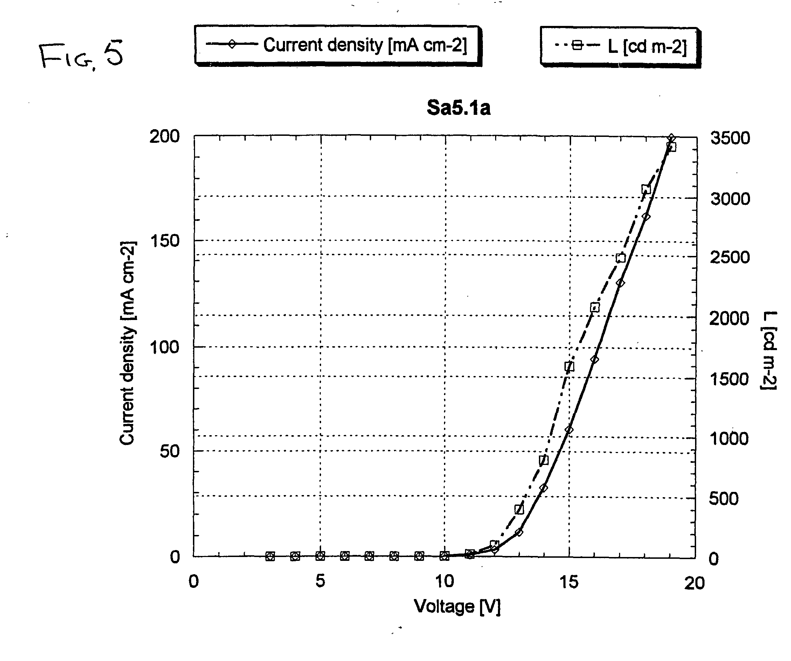

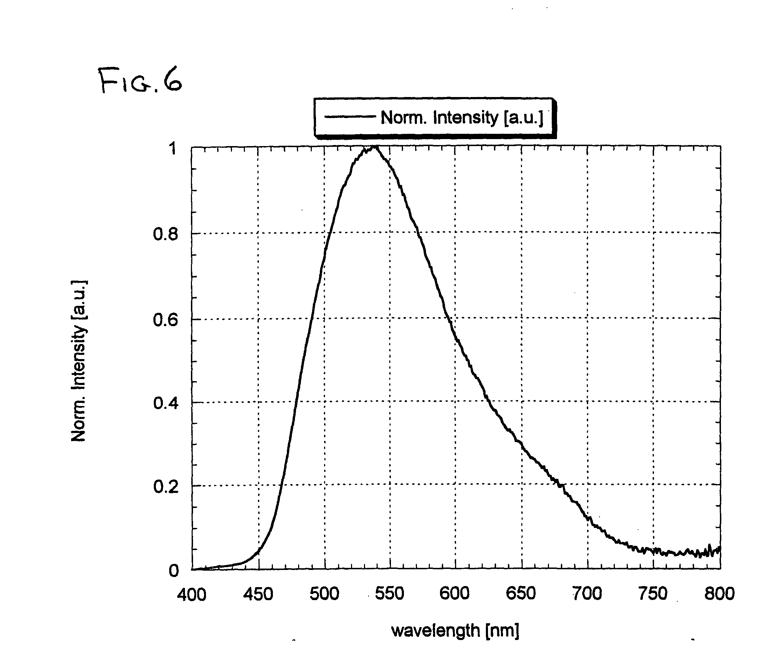

Referring now to Figs. 5 and 6, the device used to generate the data given is a three layer device where the benzofuran trimer is sandwiched between conventional hole and electron transport materials. The light output of the device was 3340 cdm⁻² at 19V and 200 mAcm⁻². The CIE co-ordinates were x=0.32 and y=0.52. In this example, the emission is from the Alq₃ electron transport layer.

In the above-mentioned devices, the benzofuran compound according to the present invention was used to form the layer. However, it is within the scope of the present invention to incorporate the benzofuran, benzothiophene or indole compounds according to the present invention in a host matrix in any appropriate concentration, but preferably at low concentrations (typically less than 5 % by weight of the host matrix). At low concentrations, the benzofuran, benzothiophenes and/or indoles will not aggregate with one another and so their emission properties are expected to be as good as those observed in solution. Alternatively, it is within the scope of the present invention to use one or more compounds according to the present invention as host materials and dope them with more emissive dyes for better emission.

It is considered that the compounds according to the present invention have the potential to be charge transport materials, particularly hole transport materials, or dopants for good blue emission.

With regard to the combinatorial or fast parallel synthesis of benzofuran, benzothiophene or indole compounds according to the present invention, this is potentially a useful route to enable the preparation of a large number of benzofuran, benzothiophene or indole variants quickly. Each building block added during formation of the phenylene ethynylene backbone may have a different R group appended. As noted above, there are two different benzofuran, benzothiophene or indole geometries which may be used, and the length of the oligomer/polymer may also be varied.

## Claims

1. A compound which is a substituted or unsubstituted benzofuran, benzothiophene or indole oligomer, co-oligomer, polymer or co-polymer and which includes a unit of the general formula (I): 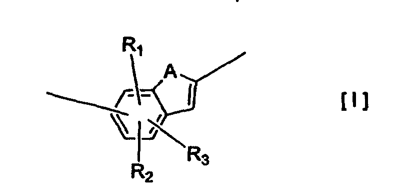 wherein A is O, S or NH, and each of R₁, R₂ and R₃ is independently selected from hydrogen, an aliphatic substituent, and an aromatic substituent.

2. A compound as claimed in claim 1 where the unit of the formula (I) recurs at least twice in the compound.

3. A compound as claimed in claim 1, which is an oligomer and in which the unit of the general formula (I) recurs 2 to 4 times.

4. A compound which is a benzofuran, benzothiophene or indole cooligomer or co-polymer including the structure (II):- 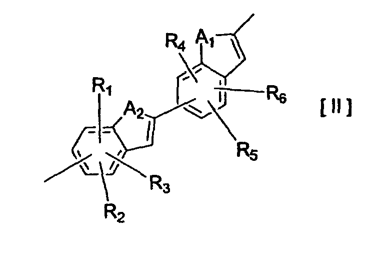 wherein A₁ and A₂ are independently selected from O, S and NH, and each of R₁, R₂, R₃, R₄, R₅ and R₆ is independently selected from hydrogen, an aliphatic substituent, and an aromatic substituent, provided that, when A₁ and A₂ are the same, not all of R₄, R₅ and R₆ are the same substituents as R₁, R₂ and R₃ and in the same respective positions as R₁, R₂ and R₃.

5. A compound as claimed in claim 1 - 4, wherein the aliphatic substituent is a substituted or unsubstituted alkyl group.

6. A compound as claimed in claim 5, wherein the alkyl group is selected from C₄ to C₆ alkyl.

7. A compound as claimed in claim 6, wherein the alkyl group is selected from hexyl and tert-butyl.

8. A compound as claimed in any preceding claim, which is hydrogen terminated at one or both ends.

9. A compound as claimed in any one of claims 1 to 7 which has a substituent terminal group at at least one end.

10. A compound as claimed in claim 9, which has an unsubstituted benzofuran, benzothiophene or indole moiety at at least one of its ends.

11. A compound as claimed in claim 9 or 10, which has a phenyl group at at least one of its ends.

12. The use of a benzofuran, benzothiophene or indole oligomer, cooligomer, polymer or co-polymer as claimed in any preceding claim in a charge transport region or an electroluminescent region of an electronic device.

13. An electroluminescent structure including a charge transport or electroluminescent region containing a benzofuran, benzothiophene or indole oligomer, co-oligomer, polymer or co-polymer as claimed in any one of claims 1 to 11.

14. An electronic device containing a benzofuran, benzothiophene or indole oligomer, co-oligomer, polymer or copolymer as claimed in any one of claims 1 to 11 as a charge transport or photoemission material.

15. A method of producing at least one benzofuran, benzothiophene or indole compound as claimed in any one of claims 1 to 11, comprising the steps of:-
(a) oligomerising, co-oligomerising, polymerising or copolymerising one or more blocked carbonyloxy-, carbonylthio- or carbonylamino-substituted phenylene ethynylene reactants;
(b) de-blocking the carbonyloxy , carbonylthio or carbonylamino groups; and
(c) effecting ring closure via the de-blocked carbonyloxy , carbonylthio or carbonylamino groups to result in formation of a furan, thiophene or pyrrole moiety.

16. A method as claimed in claim 15, when carried out by combinatorial synthesis to produce a plurality of different benzofuran, benzothiophene or indole compounds as claimed in any one of claims 1 to 11.

17. A method as claimed in claim 15 or 16, wherein the oligomerising, cooligomerising, polymerising or copolymerising step is conducted to produce a precusor compound which is soluble in a solvent, the precursor compound is dissolved in the solvent, applied to a substrate to form a thin film, and subjected to the de-blocking and ring closure steps (b) and (c) to form a relatively insoluble oligomer, co-oligomer, polymer or copolymer.
